(19) Europäisches Patentamt
European Patent Office
Office européen des brevets

(11) **EP 4 166 574 A1**

(12) **EUROPEAN PATENT APPLICATION**
published in accordance with Art. 153(4) EPC

(43) Date of publication:
**19.04.2023 Bulletin 2023/16**

(21) Application number: **21821166.2**

(22) Date of filing: **09.06.2021**

(51) International Patent Classification (IPC):
*C07K 19/00* (2006.01)    *A61K 38/22* (2006.01)
*A61K 47/68* (2017.01)    *A61P 7/06* (2006.01)
*C12N 15/12* (2006.01)    *C12N 15/13* (2006.01)
*C12N 15/62* (2006.01)    *C12P 21/02* (2006.01)

(52) Cooperative Patent Classification (CPC):
**A61K 38/22; A61K 47/68; A61P 7/06;
C07K 14/435; C07K 16/00; C07K 19/00;
C12N 15/62; C12P 21/02**

(86) International application number:
**PCT/JP2021/021972**

(87) International publication number:
**WO 2021/251438 (16.12.2021 Gazette 2021/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.06.2020  JP 2020101237**

(71) Applicant: **Bica Therapeutics Inc.
Toyonaka-shi, Osaka 560-0082 (JP)**

(72) Inventors:
• **NAKAO, Ryota
Toyonaka-shi, Osaka 560-0082 (JP)**
• **IZUMI, Yoshikatsu
Toyonaka-shi, Osaka 560-0082 (JP)**
• **HATTORI, Kunihiro
Toyonaka-shi, Osaka 560-0082 (JP)**

(74) Representative: **Heubeck, Christian
Weickmann & Weickmann
Patent- und Rechtsanwälte PartmbB
Postfach 860 820
81635 München (DE)**

Remarks:
The complete document including Reference
Table(s) and the Sequence Listing(s) can be
downloaded from the EPO website

(54) **FUSION PROTEIN CONTAINING ERYTHROPOIETIN POLYPEPTIDE**

(57)    The present invention relates to a fusion protein containing an erythropoietin polypeptide fused to the Fc region of mammal-derived IgG (hereinafter to be also abbreviated as EPO-Fc fusion protein). The fusion protein in which erythropoietin polypeptide has the amino acid sequence shown in SEQ ID NO: 9 or 10, and the Fc region has the amino acid sequence shown in SEQ ID NO: 3 or 4 is more superior in the property and activity and can be obtained at a high yield.

EP 4 166 574 A1

**Description**

[Technical Field]

The present invention relates to a fusion protein containing an erythropoietin polypeptide fused to the Fc region of dog or cat-derived IgG.

[Background Art]

Erythropoietin (hereinafter also abbreviated as EPO) is a glycoprotein with a molecular weight of 30,000-34,000 and is a factor that promotes the generation and differentiation of erythrocytes. It is produced in the kidney and is essential for regulating cell levels of erythrocytes in circulation. This protein binds to the receptor of erythrocyte progenitor cells and acts to cause an increase in intracellular calcium ion concentration, an increase in DNA biosynthesis, stimulation of hemoglobin production, and the like. Erythropoietin can be used to treat hematopoietic diseases or hematopoietic failure, but has the disadvantage of a short half-life in blood.

On the other hand, approaches have been made to bind immunoglobulin fragments to erythropoietin polypeptide because it is known that fusion of an immunoglobulin constant region to a non-immunoglobulin protein markedly prolongs the half-life of the aforementioned non-immunoglobulin protein. For example, Patent Literature 1 describes the production and use of a fusion protein containing the Fc portion of an immunoglobulin and an erythropoietin polypeptide. In fact, it has been reported that a fusion protein containing the Fc portion of an immunoglobulin and an erythropoietin polypeptide extended the half-life of the erythropoietin polypeptide in vivo.

Neonatal Fc receptor (hereinafter to be also referred to as FcRn) avoids lysosomal degradation of IgG by binding to the Fc region of IgG and recycling same into plasma. IgG shows prolonged retention in plasma by binding to FcRn. Binding of IgG to FcRn is observed only under acidic conditions (e.g., pH 6.0), and the binding is scarcely observed under neutral conditions (e.g., pH 7.4). Generally, IgG is non-specifically uptaken into cells via endocytosis. It returns to the cell surface by binding to FcRn in endosome under acidic conditions in the endosome and is recycled by dissociating from FcRn under neutral conditions in plasma, resulting in longer retention in plasma than other plasma proteins. IgG that did not bind to FcRn in endosome moves to lysosome where it is degraded.

As a method for improving retention of IgG in plasma, in human, a method for improving the binding ability to FcRn under acidic conditions has been reported. By increasing the binding ability to FcRn under acidic conditions by introducing amino acid substitution into the Fc region of IgG, the recycling efficiency from endosome to plasma increases, thus resulting in improved retention in plasma (patent documents 2, 3, non-patent documents 1 - 3).

In addition, cytokines or soluble membrane receptors, which is fused with Fc region of IgG, and the like (Fc fusion proteins) have been developed as therapeutic pharmaceutical products for human. These achieve long retention in plasma through binding to FcRn, like IgG.

As described above, biopharmaceutical products have been developed for humans by modifying and applying the binding between the Fc region of IgG and FcRn, and the development of biopharmaceutical products with similarly-improved retention in plasma has been desired for animals other than human, such as dog, cat, and the like.

However, alteration of amino acids in the Fc region that improves and advances retention of antibodies in plasma in dogs and cats is not known.

[Citation List]

[Patent Literature]

[PTL 1]
WO 99/02709
[PTL 2]
WO 2002/060919
[PTL 3]
WO 2012/083370

[Non Patent Literature]

[NPL 1]
Yeung YA et al., J. Immunol. (2009) 182, 7663-71.
[NPL 2]
Datta-Mannan A et al., J. Biol. Chem. (2007) 282, 1709-17.
[NPL 3]
Dall'Acqua WF et al., J. Immunol. (2002) 169, 5171-80.

[Summary of Invention]

[Technical Problem]

The present invention aims to acquire a fusion protein containing an erythropoietin polypeptide fused to the Fc region of IgG and more superior in the property, activity, and plasma retention (hereinafter to be also abbreviated as EPO-Fc fusion protein) in a high yield.

[Solution to Problem]

In order to solve the above-mentioned problem, the present inventors added various amino acid alterations to an EPO portion, an Fc region, and a hinge region (when the hinge region interlies between the EPO portion and the Fc region) of the EPO-Fc fusion protein, and examined the effects thereof. As a result, it was found that an EPO-Fc fusion protein more superior in the physical property, activity, and yield can be obtained by applying a specific mutation to the EPO region and hinge region. Furthermore, the present inventors have found that an EPO-Fc fusion protein more superior in the plasma retention in addition to the physical property, activity, and yield can be obtained by using, as the Fc region, an Fc region having an amino acid alteration at a specific position (so-called Fc region variant) with improved binding ability to FcRn under acidic conditions and improved plasma retention, and completed the present invention.

Accordingly, the present invention provides the following.

[1] A fusion protein comprising an erythropoietin polypeptide fused to the Fc region of a dog or cat-derived IgG.

[2] The fusion protein of the above-mentioned [1], wherein the erythropoietin polypeptide is fused to the Fc region via a hinge region of the IgG.

[3] The fusion protein of the above-mentioned [1] or [2], wherein the erythropoietin polypeptide has at least one mutation, and the mutation is substitution of at least one cysteine in a wild-type sequence thereof with arginine or proline.

[4] The fusion protein of the above-mentioned [3], wherein the erythropoietin polypeptide having at least one mutation is derived from cat, and has the amino acid sequence shown in SEQ ID NO: 9 or 10.

[5] The fusion protein of any of the above-mentioned [2] to [4], wherein the hinge region has at least one mutation, and the mutation is substitution of at least one cysteine in a wild-type sequence thereof with glycine.

[6] The fusion protein of the above-mentioned [5], wherein the hinge region having at least one mutation is derived from cat, and has the amino acid sequence shown in any of SEQ ID NO: 5 - 8.

[7] The fusion protein of any of the above-mentioned [1] to [6], wherein the Fc region has an amino acid alteration.

[8] The fusion protein of the above-mentioned [7], wherein the Fc region is derived from dog IgG.

[9] The fusion protein of the above-mentioned [7], wherein the Fc region is derived from cat IgG.

[10] The fusion protein of the above-mentioned [8], wherein the amino acid alteration in the Fc region comprises at least one selected from the group consisting of

(i) substitution of the 252-position leucine with tyrosine or threonine,
(ii) substitution of the 254-position alanine with threonine,
(iii) substitution of the 256-position threonine with glutamic acid,
(iv) substitution of the 308-position isoleucine with proline,
(v) substitution of the 428-position methionine with leucine,
(vi) substitution of the 433-position histidine with leucine,
(vii) substitution of the 434-position asparagine with alanine, serine, tyrosine or phenylalanine,
(viii) substitution of the 436-position tyrosine with threonine,
(ix) substitution of the 438-position glutamine with arginine, and
(x) substitution of the 440-position serine with glutamic acid (wherein the numbering of amino acids in the Fc region is based on EU Index of Kabat using Fc region of human antibody as the standard).

[11] The fusion protein of the above-mentioned [10], wherein the amino acid alterations in the Fc region are

(i) substitution of the 434-position asparagine with alanine,
(ii) substitution of the 436-position tyrosine with threonine,
(iii) substitution of the 438-position glutamine with arginine, and
(iv) substitution of the 440-position serine with glutamic acid.

[12] The fusion protein of the above-mentioned [10], wherein the amino acid alterations in the Fc region are

(i) substitution of the 428-position methionine with leucine,
(ii) substitution of the 434-position asparagine with alanine,
(iii) substitution of the 436-position tyrosine with threonine,
(iv) substitution of the 438-position glutamine with arginine, and
(v) substitution of the 440-position serine with glutamic acid.

[13] The fusion protein of the above-mentioned [10], wherein the amino acid alteration in the Fc region are

(i) substitution of the 428-position methionine with leucine,
(ii) substitution of the 434-position asparagine with alanine,
(iii) substitution of the 438-position glutamine with arginine, and
(iv) substitution of the 440-position serine with glutamic acid.

[14] The fusion protein of the above-mentioned [10], wherein the amino acid alteration in the Fc region are

(i) substitution of the 252-position leucine with tyrosine,
(ii) substitution of the 254-position alanine with threonine, and
(iii) substitution of the 256-position threonine with glutamic acid.

[15] The fusion protein of the above-mentioned [10], wherein the amino acid alteration in the Fc region are

(i) substitution of the 428-position methionine with leucine, and
(ii) substitution of the 434-position asparagine with serine.

[16] The fusion protein of the above-mentioned [10], wherein the amino acid alteration in the Fc region are

(i) substitution of the 308-position isoleucine with proline, and
(ii) substitution of the 434-position asparagine with tyrosine.

[17] The fusion protein of the above-mentioned [10], wherein the amino acid alteration in the Fc region are

(i) substitution of the 252-position leucine with threonine,
(ii) substitution of the 254-position alanine with threonine,
(iii) substitution of the 256-position threonine with glutamic acid,
(iv) substitution of the 433-position histidine with leucine, and
(v) substitution of the 434-position asparagine with phenylalanine.

[18] The fusion protein of the above-mentioned [9], wherein the amino acid alteration in the Fc region includes at least one selected from the group consisting of

(i) substitution of the 252-position serine with tyrosine or threonine,
(ii) substitution of the 254-position serine with threonine,
(iii) substitution of the 256-position threonine with glutamic acid,
(iv) substitution of the 259-position valine with isoleucine,
(v) substitution of the 308-position isoleucine with proline or phenylalanine,
(vi) substitution of the 428-position serine with leucine,
(vii) substitution of the 433-position histidine with leucine,
(viii) substitution of the 434-position serine with alanine, tyrosine or phenylalanine,
(ix) substitution of the 436-position histidine with threonine,
(x) substitution of the 438-position glutamine with arginine,
(xi) substitution of the 440-position serine with glutamic acid,

(xii) substitution of the 235-position leucine with arginine,
(xiii) substitution of the 236-position glycine with arginine, and
(xiv) substitution of the 239-position serine with lysine (wherein the numbering of amino acids in the Fc region is based on EU Index of Kabat using Fc region of human antibody as the standard).

[19] The fusion protein of the above-mentioned [18], wherein the amino acid alterations in the Fc region are

(i) substitution of the 434-position serine with alanine,
(ii) substitution of the 436-position histidine with threonine,
(iii) substitution of the 438-position glutamine with arginine, and
(iv) substitution of the 440-position serine with glutamic acid.

[20] The fusion protein of the above-mentioned [18], wherein the amino acid alterations in the Fc region are

(i) substitution of the 428-position serine with leucine,
(ii) substitution of the 434-position serine with alanine,
(iii) substitution of the 436-position histidine with threonine,
(iv) substitution of the 438-position glutamine with arginine, and
(v) substitution of the 440-position serine with glutamic acid.

[21] The fusion protein of the above-mentioned [18], wherein the amino acid alterations in the Fc region are

(i) substitution of the 428-position serine with leucine,
(ii) substitution of the 434-position serine with alanine,
(iii) substitution of the 438-position glutamine with arginine, and
(iv) substitution of the 440-position serine with glutamic acid.

[22] The fusion protein of the above-mentioned [18], wherein the amino acid alterations in the Fc region are

(i) substitution of the 252-position serine with tyrosine,
(ii) substitution of the 254-position serine with threonine, and
(iii) substitution of the 256-position threonine with glutamic acid.

[23] The fusion protein of the above-mentioned [18], wherein the amino acid alterations in the Fc region are

(i) substitution of the 308-position isoleucine with proline, and
(ii) substitution of the 434-position serine with tyrosine.

[24] The fusion protein of the above-mentioned [18], wherein the amino acid alterations in the Fc region are

(i) substitution of the 259-position valine with isoleucine,
(ii) substitution of the 308-position isoleucine with phenylalanine, and
(iii) substitution of the 428-position serine with leucine.

[25] The fusion protein of the above-mentioned [18], wherein the amino acid alterations in the Fc region are

(i) substitution of the 252-position serine with threonine,
(ii) substitution of the 254-position serine with threonine,
(iii) substitution of the 256-position threonine with glutamic acid,
(iv) substitution of the 433-position histidine with leucine, and
(v) substitution of the 434-position serine with phenylalanine.

[26] The fusion protein of the above-mentioned [18], wherein the amino acid alterations in the Fc region are

(vi) substitution of the 428-position serine with leucine,
(viii) substitution of the 434-position serine with alanine,
(x) substitution of the 438-position glutamine with arginine,
(xi) substitution of the 440-position serine with glutamic acid,

(xii) substitution of the 235-position leucine with arginine,
(xiii) substitution of the 236-position glycine with arginine, and
(xiv) substitution of the 239-position serine with lysine.

[27] The fusion protein of the above-mentioned [7], wherein the Fc region of IgG having the amino acid alteration is derived from cat, and has the amino acid sequence shown in SEQ ID NO: 3 or 4.

[28] A pharmaceutical composition comprising the fusion protein of any of the above-mentioned [1] to [27].

[29] The pharmaceutical composition of the above-mentioned [28] for the treatment of a hematopoiesis disease or hematopoiesis failure.

[Advantageous Effects of Invention]

The EPO-Fc fusion protein of the present invention is superior in the property and activity. According to the present invention, the EPO-Fc fusion protein can be obtained at a high yield.

[Brief Description of Drawings]

[Fig. 1]

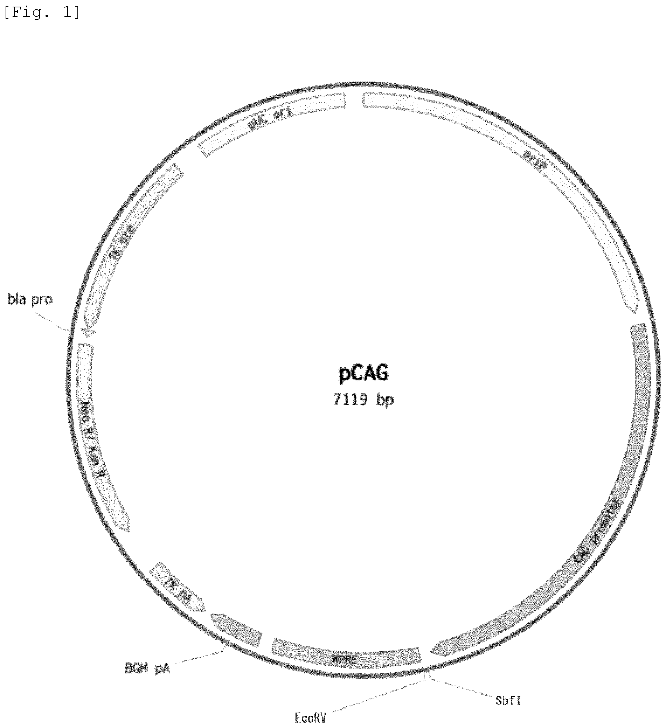

Fig. 1 is a plasmid map showing the structure of pCAG.
[Fig. 2]

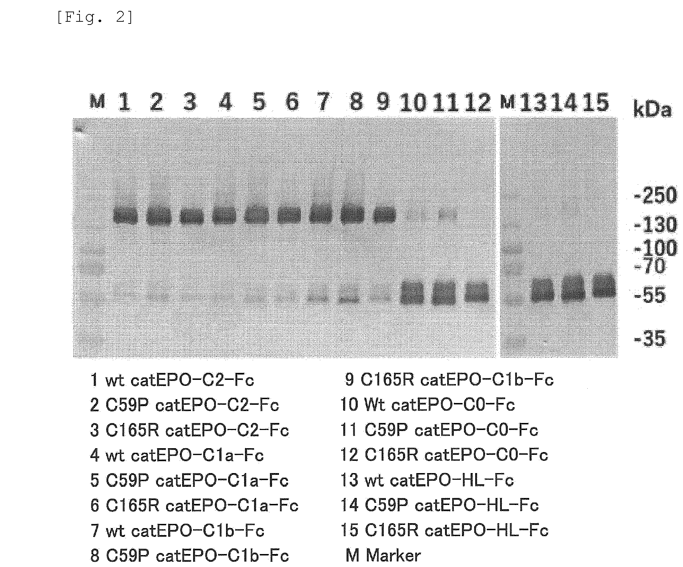

Fig. 2 is a view showing the results of Western blot analysis of the EPO region- and hinge region-altered cat EPO-Fc fusion proteins.
[Fig. 3]

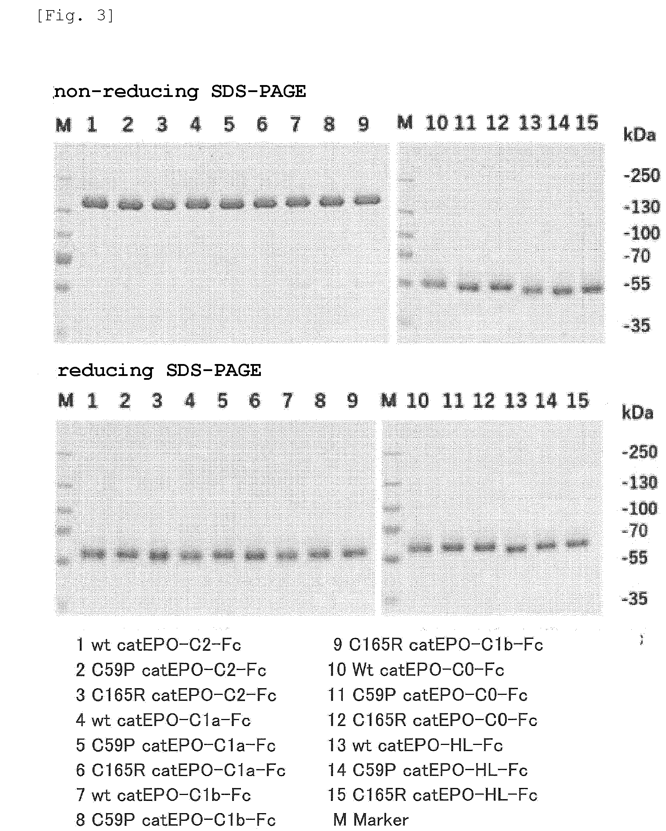

Fig. 3 shows views confirming the presence or absence of intermolecular disulfide bonds in cat EPO-Fc by SDS-PAGE under reducing and non-reducing conditions and CBB staining.
[Fig. 4A]

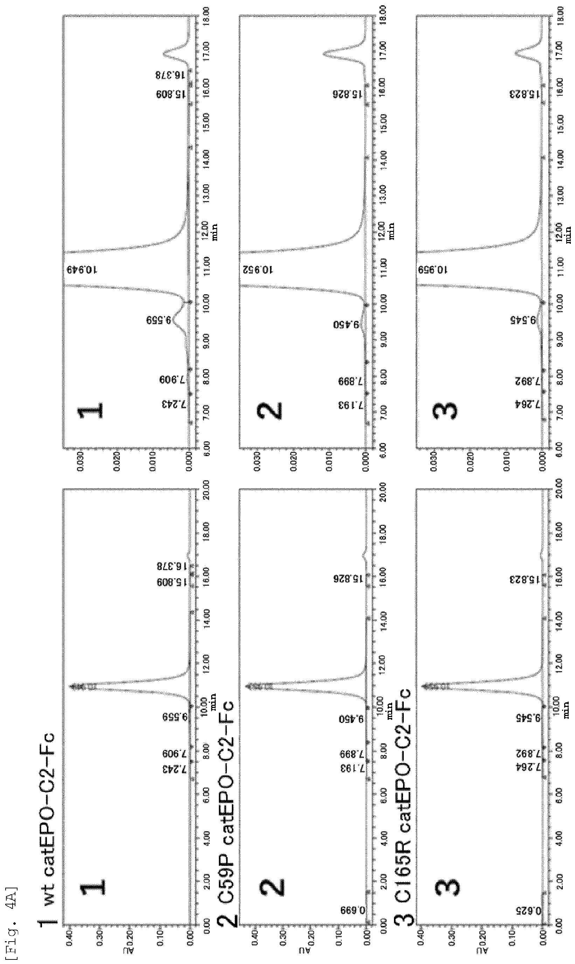

Fig. 4A shows views exhibiting SEC analysis chromatograms of EPO region-, hinge region-altered cat EPO-Fc fusion proteins (1 to 3 in Table 8). The views on the right are enlarged views of the views on the left.
[Fig. 4B]

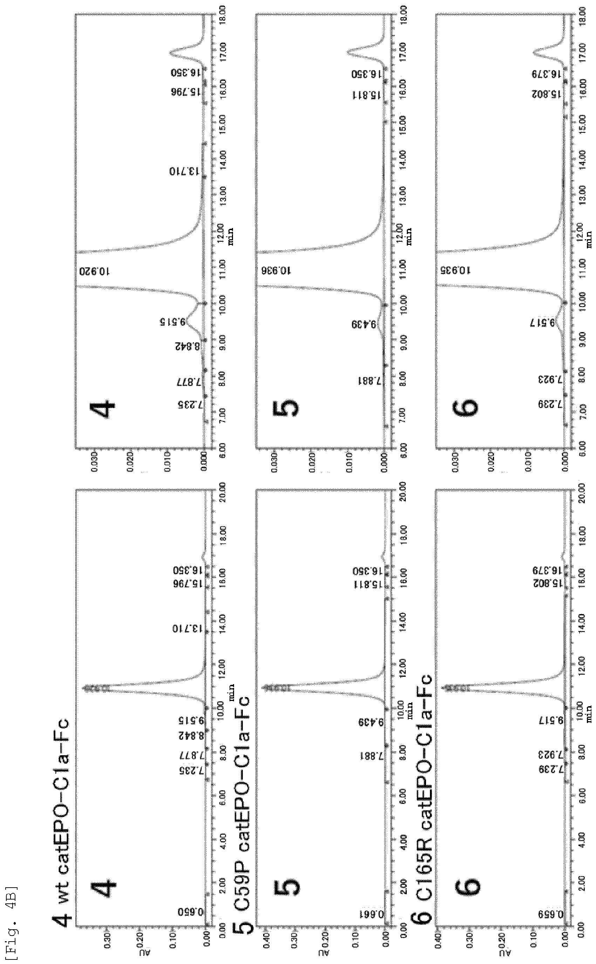

Fig. 4B shows views exhibiting SEC analysis chromatograms of EPO region-, hinge region-altered cat EPO-Fc fusion proteins (4 to 6 in Table 8). The views on the right are enlarged views of the views on the left.
[Fig. 4C]

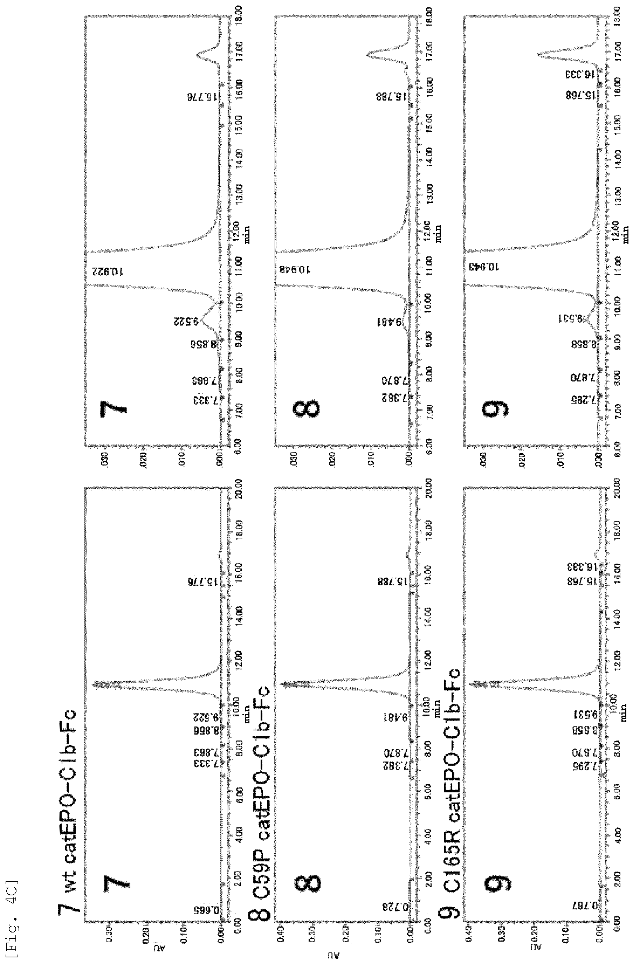

Fig. 4C shows views exhibiting SEC analysis chromatograms of EPO region-, hinge region-altered cat EPO-Fc fusion proteins (7 to 9 in Table 8). The views on the right are enlarged views of the views on the left.
[Fig. 4D]

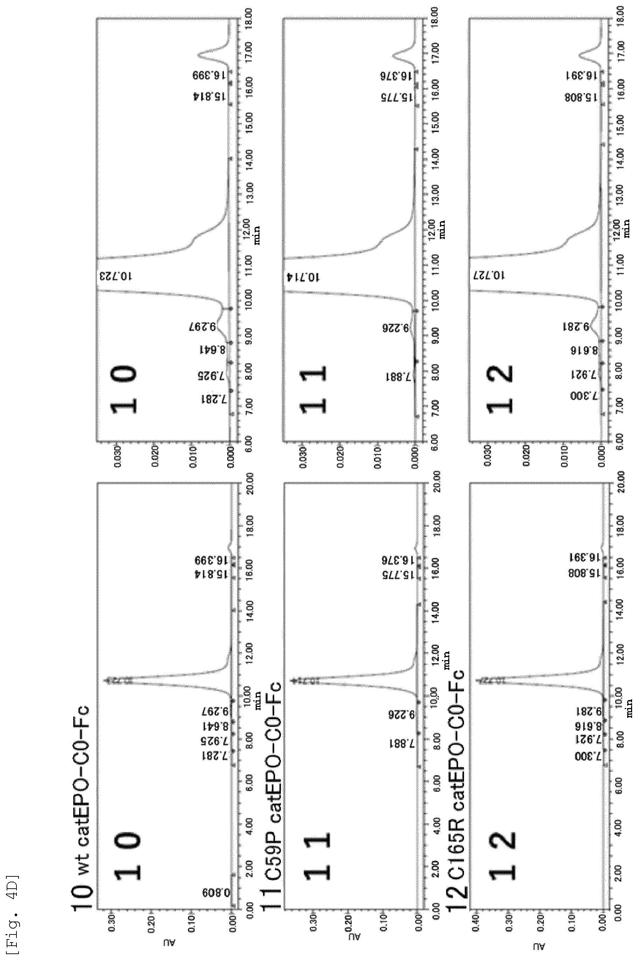

Fig. 4D shows views exhibiting SEC analysis chromatograms of EPO region-, hinge region-altered cat EPO-Fc fusion proteins (10 to 12 in Table 8). The views on the right are enlarged views of the views on the left.
[Fig. 4E]

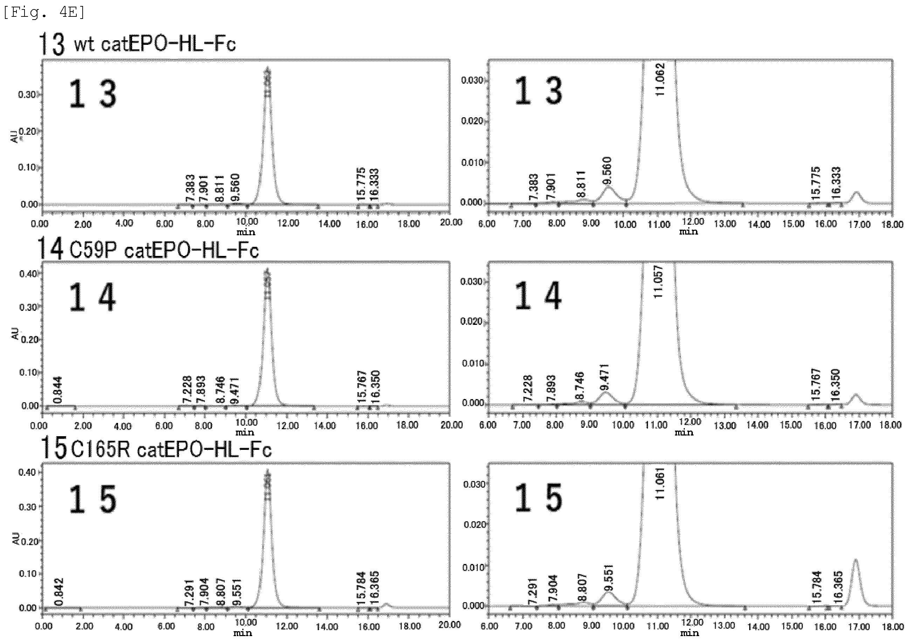

Fig. 4E shows views exhibiting SEC analysis chromatograms of EPO region-, hinge region-altered cat EPO-Fc fusion proteins (13 to 15 in Table 8). The views on the right are enlarged views of the views on the left.
[Fig. 4F]

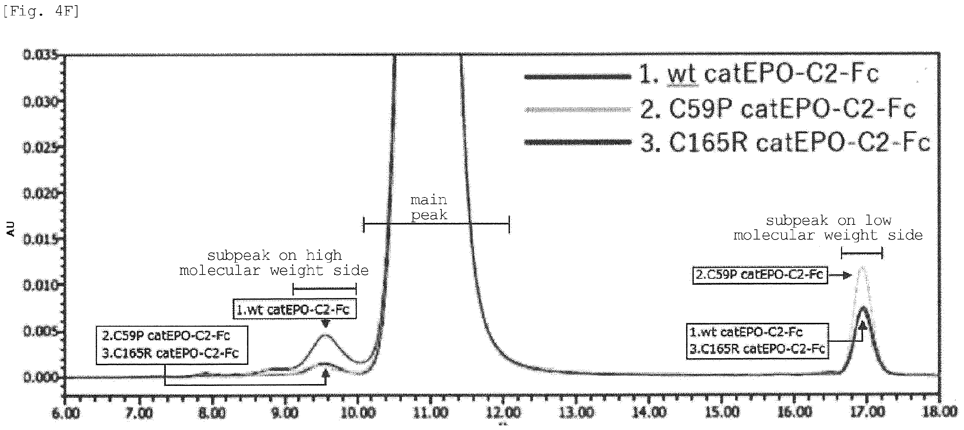

Fig. 4F shows a chromatogram merging the results of SEC analysis of cat EPO-Fc fusion proteins, wt cat EPO-C2-Fc, C59P cat EPO-C2-Fc, and C165R cat EPO-C2-Fc (1 to 3 in Table 8).
[Fig. 5]

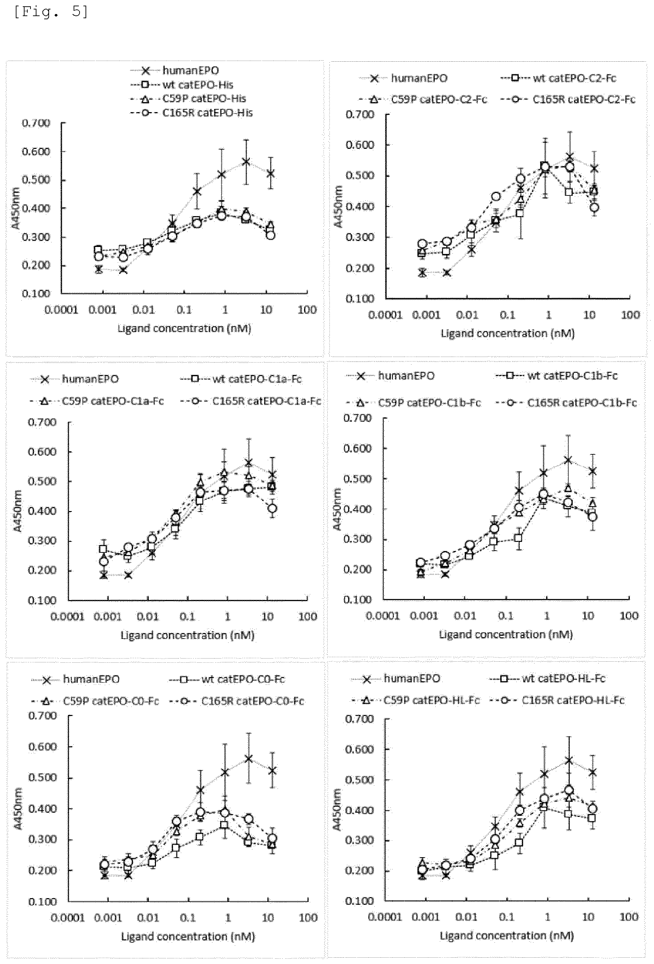

Fig. 5 shows graphs of the results of bioactivity evaluation of cat EPO-Fc fusion proteins using the proliferation of TF-1 cells as an index.
[Fig. 6A]

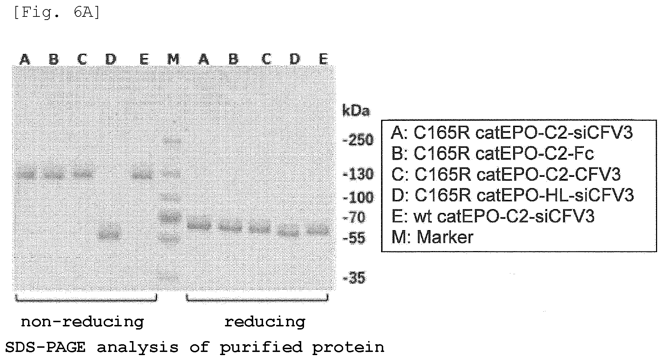

Fig. 6A shows the results of SDS-PAGE analysis of purified cat EPO-Fc fusion proteins.
[Fig. 6B]

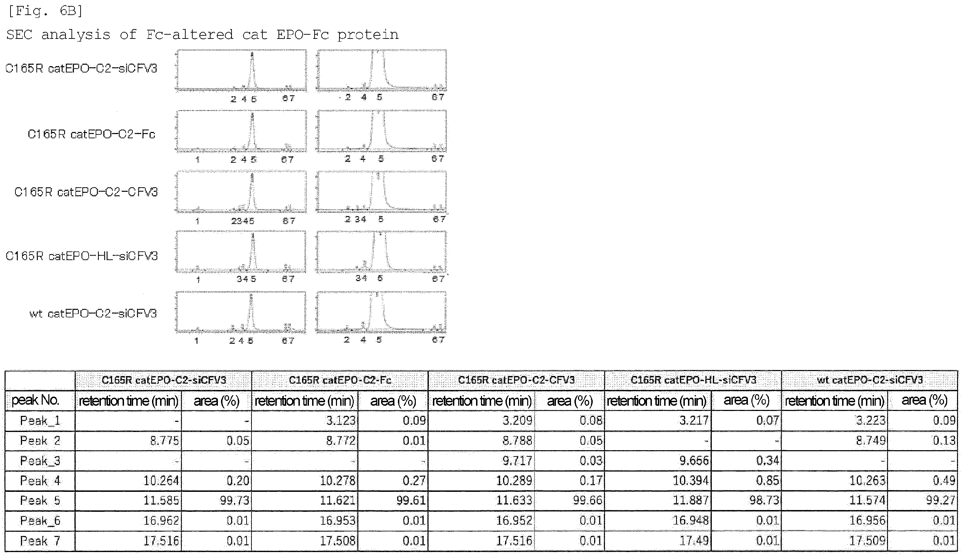

Fig. 6B shows the results of SEC analysis of purified cat EPO-Fc fusion proteins (lower panel). The SEC analysis chromatograms (upper panel) were digitized.
[Fig. 7]

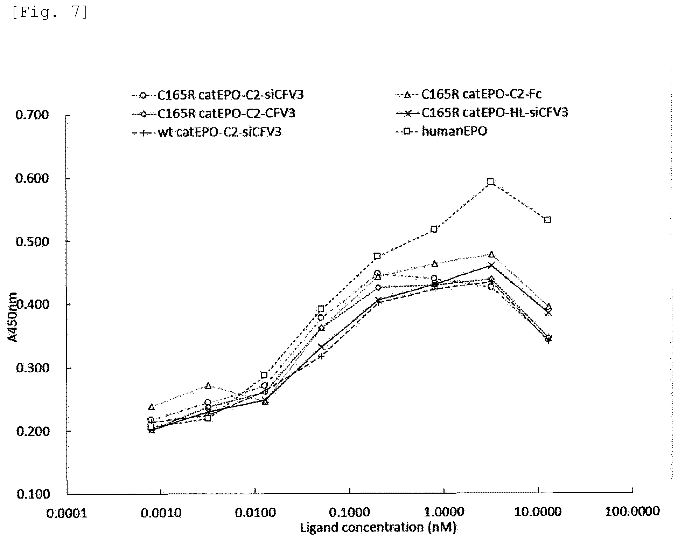

Fig. 7 is a graph showing the results of in vitro activity evaluation of Fc-altered cat EPO-Fc fusion proteins using the proliferation of TF-1 cells as an index.
[Fig. 8]

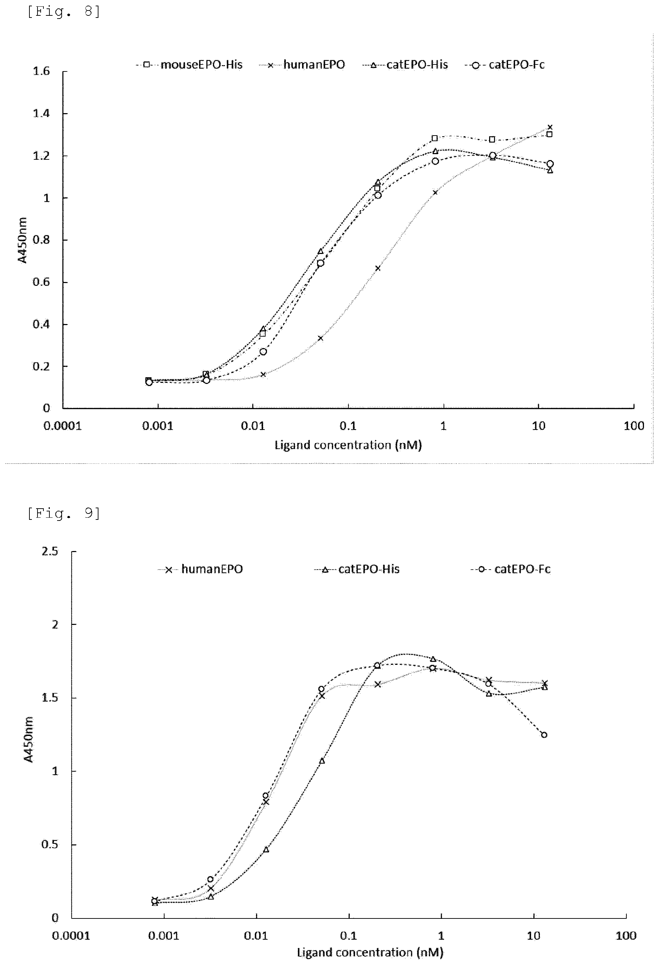

Fig. 8 is a graph showing the results of in vitro activity evaluation of Fc-altered cat EPO-Fc fusion proteins using the proliferation of BaF3/mouse EPOR cells as an index.
[Fig. 9]
Fig. 9 is a graph showing the results of in vitro activity evaluation of Fc-altered cat EPO-Fc fusion proteins using the proliferation of BaF3/cat EPOR cells as an index.

[Description of Embodiments]

The present invention provides a fusion protein containing an EPO polypeptide bound to the Fc region of IgG derived from a mammal (hereinafter to be also referred to as EPO-Fc fusion protein). The EPO-Fc fusion protein of the present invention is explained in detail below.

1. EPO-Fc fusion protein

In the present specification, the "EPO-Fc fusion protein" means a protein containing the Fc region of dog or cat-derived IgG and an erythropoietin polypeptide. In a preferred embodiment of the present invention, the EPO-Fc fusion protein forms a homo dimer, and thus generally has one or two or more disulfide bonds.
The mode of binding between the Fc region and the EPO polypeptide is not particularly limited as long as the two are functionally linked. In one embodiment, the EPO polypeptide is directly linked to the Fc region via a covalent bond. In another embodiment, the EPO polypeptide is indirectly linked to the Fc region. For example, in an EPO-Fc fusion protein, a linker can be included between the Fc region and the erythropoietin polypeptide.

2. Fc region

In the present invention, the "Fc region" includes a domain derived from the constant region of dog or cat IgG, preferably cat IgG, and this includes fragments and variants of the constant region.
IgG includes isoforms, and the number thereof varies depending on the animal species. In human, mouse and rat, 4 types of IgG1 to IgG4 are known. There are also four IgG immunoglobulins in dog, and these are defined as caIgG-A, caIgG-B, caIgG-C and caIgG-D (Tang et al., Vet. Immunol. Immunopathol. 80(3-4), 259-270, 2001). In cat, there are three types of IgG immunoglobulins, the presence of which as IgG1a, IgG1b, and IgG2 has been reported.

1) Kanai, T.H., et al., 2000. Identification of two allelic IgG1 C(H)coding regions (Cgamma1) of cat. Vet. Immunol. Immunopathol. 73(1), 53-62.
2) Strietzel, C.J., et al., 2014. In Vitro functional characterization of feline IgGs, Vet. Immunol. Immunopathol. 158 (3-4), 214-233.

The constant region of an immunoglobulin is defined as a naturally or synthetically produced polypeptide homologous to an immunoglobulin C-terminal region. It can contain CH1 domain, hinge, CH2 domain, CH3 domain, or CH4 domain, individually or in any combination. The Fc region of the heavy chain consists of CH2 domain and CH3 domain. The hinge region is located between CH1 domain and CH2 domain.
In the present invention, the Fc region of dog or cat IgG may preferably be an altered Fc region in which amino acid alterations are introduced into the Fc region of wild-type dog or cat IgG. It is more preferably an altered Fc region showing higher activity of binding to dog or cat FcRn (hereinafter also referred to as FcRn-binding activity) under acidic conditions than the FcRn-binding activity of the parent peptide before alteration, and having at least one amino acid alteration.
"FcRn" is structurally similar to a major histocompatibility complex (MHC) class I polypeptide and has 22 to 29% sequence identity with class I MHC molecule in human (reference document for human: Ghetie et al., Immunol. Today (1997) 18 (12), 592-598).
FcRn is expressed as a heterodimer consisting of a soluble β-chain (or light chain) β2-microglobulin (sometimes indicated as β2m) and a transmembrane α-chain (or heavy chain, sometimes indicated as FCGRT). The α chain of FcRn consists of three extracellular domains (α1, α2, α3), and the α1 and α2 domains interact with the FcRn binding domain in the Fc region of antibody (Raghavan et al., Immunity (1994) 1, 303-315).
FcRn forms a complex with in vivo β2-microglobulin. A complex of soluble FcRn with β2-microglobulin is prepared using a conventional recombinant expression method (see "Preparation of FcRn Expression Vector" and "Expression and Purification of FcRn Protein" in Example), and the complex can be used for evaluation of the FcRn binding activity in the present invention. In the present invention, unless otherwise specified, FcRn is used as a complex with β2-microglobulin.
The "parent polypeptide" means a polypeptide before introduction of the amino acid alteration as opposed to the polypeptide after introduction of the alteration. Examples of the parent polypeptide containing the Fc region of dog or cat IgG include a polypeptide containing the Fc region of natural IgG of dog or cat, and preferred is an antibody, particularly a polypeptide constituting the natural IgG of dog or cat. A polypeptide having an Fc region in which an amino acid alteration is introduced into the Fc region of the parent polypeptide is also referred to as an Fc region variant.

The wild-type IgG of dog or cat means a polypeptide that contains the same amino acid sequence as naturally-occurring IgG of dog or cat and belongs to the class of antibody substantially encoded by an immunoglobulin gamma gene.

Examples of the amino acid alteration in the Fc region include substitution, insertion, deletion, and the like of amino acids, preferably substitution of amino acids. The number of amino acids to be altered is not particularly limited, and only one amino acid may be altered, or two or more amino acids may be altered. Amino acids at two to several positions, more preferably 2 to 5 positions, are preferably altered.

In the case of dog or cat, the following alteration is preferred.

In the present specification, the alphabet displayed on the left side of the number representing the number of amino acid residues up to the substitution site indicates one-letter notation of the amino acid before substitution, and the alphabet displayed on the right side indicates one-letter notation of the amino acid after substitution. The number of amino acid residues up to the substitution site is shown by the EU numbering system of Kabat in the Fc region of human IgG that has been adapted to the Fc region of dog or cat. The "EU numbering system" or "EU Index" is generally used to refer to residues in the heavy chain constant region of an antibody (e.g., Kabat et al., Sequences of Proteins of Immunological Interest. 5th Ed. Public Health Service, National Institutes of Health, Bethesda, Md. (1991)). The "EU numbering system of Kabat" means residue numbering for human IgG1 EU antibody. Unless otherwise specified in the present specification, references to residue numbers is based on the EU numbering of Kabat that has been adapted to the sequences of dog or cat.

(In the case of dog)

(i) substitution of the 252-position leucine with tyrosine or threonine (L252Y or L252T),
(ii) substitution of the 254-position alanine with threonine (A254T),
(iii) substitution of the 256-position threonine with glutamic acid (T256E),
(iv) substitution of the 308-position isoleucine with proline (I308P),
(v) substitution of the 428-position methionine with leucine (M428L),
(vi) substitution of the 433-position histidine with leucine (H433L),
(vii) substitution of the 434-position asparagine with alanine, serine, tyrosine or phenylalanine (N434A, N434S, N434Y or N434F),
(viii) substitution of the 436-position tyrosine with threonine (Y436T),
(ix) substitution of the 438-position glutamine with arginine (Q438R), and
(x) substitution of the 440-position serine with glutamic acid (S440E)

At least one, preferably two or more, of the alterations are present.

Preferable examples of the alteration include the following DFV-1 to DFV-6, DFV-8.

DFV-1; N434A, Y436T, Q438R, S440E
DFV-2; M428L, N434A, Y436T, Q438R, S440E
DFV-3; M428L, N434A, Q438R, S440E
DFV-4; L252Y, A254T, T256E
DFV-5; M428L, N434S
DFV-6; I308P, N434Y
DFV-8; L252T, A254T, T256E, H433L, N434F

(In the case of cat)

(i) substitution of the 252-position serine with tyrosine or threonine (S252Y or S252T),
(ii) substitution of the 254-position serine with threonine (S254T),
(iii) substitution of the 256-position threonine with glutamic acid (T256E),
(iv) substitution of the 259-position valine with isoleucine (V259I),
(v) substitution of the 308-position isoleucine with proline or phenylalanine (I308P or I308F),
(vi) substitution of the 428-position serine with leucine (S428L),
(vii) substitution of the 433-position histidine with leucine (H433L),
(viii) substitution of the 434-position serine with alanine, tyrosine or phenylalanine (S434A, S434Y or S434F),

(ix) substitution of the 436-position histidine with threonine (H436T),
(x) substitution of the 438-position glutamine with arginine (Q438R),
(xi) substitution of the 440-position serine with glutamic acid (S440E)
(xii) substitution of the 235-position leucine with arginine (L235R),
(xiii) substitution of the 236-position glycine with arginine (G236R), and
(xiv) substitution of the 239-position serine with lysine (S239K)

At least one, preferably two or more, of the alterations are present.

Preferable examples of the alteration include the following CFV-1 to CFV-4, CFV-6 to DFV-8, and siCFV3.

CFV-1; S434A, H436T, Q438R, S440E
CFV-2; S428L, S434A, H436T, Q438R, S440E
CFV-3; S428L, S434A, Q438R, S440E
CFV-4; S252Y, S254T, T256E
CFV-6; I308P, S434Y
CFV-7; V259I, I308F, S428L
CFV-8; S252T, S254T, T256E, H433L, S434F
siCFV-3; L235R, G236R, S239K, S428L, S434A, Q438R, S440E

Table 1 summarizes respective alterations when human EU numbering is made to correspond to the dog or cat sequence, and when the amino acid at the start of the dog or cat Fc region is 1.

[Table 1]

| "Position of dog, cat Fc alteration" | | | |
|---|---|---|---|
| dog | name of variant | alteration (when human EU numbering is adapted to dog sequences) | alteration (amino acid at the start of dog Fc region_SEQ ID NO: 1 as 1) |
| | DFV-1 | N434A/Y436T/Q438R/S440E | N206A/Y208T/Q210R/S212E |
| | DFV-2 | M428L/N434A/Y436T/Q438R/ S440E | M200L/N206A/Y208T/Q210R/ S212E |
| | DFV-3 | M428L/N434A/Q438R/S440E/ | M200L/N206A/Q210R/S212E/ |
| | DFV-4 | L252Y/A254T/T256E | L22Y/A24T/T26E |
| | DFV-5 | M428L/N434S | M200L/N206S |
| | DFV-6 | I308P/N434Y | I78P/N206Y |
| | DFV-7 | V259I/I308F/M428L | V29I/I78F/M200L |
| | DFV-8 | L252T/A254T/T256E/H433L/ N434F | L22T/A24T/T26E/H205L/ N206F |
| cat | name of variant | alteration (when human EU numbering is adapted to cat sequences) | alteration (amino acid at the start of cat Fc region_SEQ ID NO: 2 as 1) |
| | CFV-1 | S434A/H436T/Q438R/S440E | S206A/H208T/Q210R/S212E |
| | CFV-2 | S428L/S434A/H436T/Q438R/ S440E | S200L/S206A/H208T/Q210R/ S212E |
| | CFV-3 | S428L/S434A/Q438R/S440E | S200L/S206A/Q210R/S212E |
| | CFV-4 | S252Y/S254T/T256E | S22Y/S24T/T26E |
| | CFV-6 | I308P/S434Y | I78P/S206Y |
| | CFV-7 | V259I/I308F/S428L | V29I/I78F/S200L |
| | CFV-8 | S252T/S254T/T256E/H433L/ S434F | S22T/S24T/T26E/H205L/ S206F |
| | siCFV-3 | L235R/G236R/S239K/S428L/ S434A/Q438R/S440E | L5R/G6R/S9K/S200L/S206A/ Q210R/S212E |

When the EPO-Fc fusion protein of the present invention is derived from cat, the Fc region to be used is preferably CFV-3 shown in SEQ ID NO:3 or siCFV-3 shown in SEQ ID NO:4.

The polypeptide containing the Fc region of IgG to be used in the present invention may be altered to, for example, enhance ADCC (antibody-dependent-cellular-cytotoxicity) activity and CDC (complement-dependent cytotoxicity) activity, to increase protease resistance, to decrease effector function, to decrease the binding activity to complement, to improve antibody heterogeneity and stability, to accelerate the clearance of antigen, to cause repeated binding to multiple molecule antigens, to reduce the pI of the constant region for the purpose of increasing blood retention property, to have a binding ability to other antigens and the like, in addition to the binding with the EPO portion. For more details, the technique of Fc engineering described in Current Pharmaceutical Biotechnology, 2016, 17, 1298-1314 can be referred to. References to residue numbers in this literature are based on the EU numbering system of Kabat. The type of these alterations may be, for example, any of substitution, deletion, addition, insertion, and modification of amino acids, or combinations thereof, and preferred is substitution of amino acids.

Such alterations (deletion, substitution, insertion, addition) of amino acid can be introduced into an amino acid sequence by partially modifying the base sequence encoding the amino acid sequence. For this partial alteration of the base sequence, known methods such as known site-specific mutagenesis method (Site specific mutagenesis) (Proc Natl Acsd Sci USA., 1984 Vol. 81 5662-5666; Sambrook et al., Molecular Cloning A Laboratory Manual (1989) Second edition, Cold Spring Harbor Laboratory Press), Overlap extension PCR and the like can be appropriately adopted. In addition, a plurality of known methods may be adopted as a method for changing into an amino acid other than the natural amino acid (Annu. Rev. Biophys. Biomol. Struct. (2006) 35, 225-249, Proc. Natl. Acad. Sci. U.S.A. (2003) 100 (11), 6353-6357). For example, a cell-free translation system in which a complementary amber suppressor tRNA of a UAG codon (amber codon), which is one of the stop codons, contains a tRNA to which an unnatural amino acid is bound (Clover Direct (Protein Express)) and the like are preferably used.

Also, the methods for altering the Fc region of human IgG1 carried out in the following literatures can be referred to.

Drug Metab Dispos. 2007 Jan; 35(1):86-94,
Int Immunol. 2006 Dec; 18(12):1759-69,
J Biol Chem. 2001 Mar 2; 276(9):6591-604,
J Biol Chem. 2007; 282(3):1709-17,
J Immunol. 2002; 169(9):5171-80,
J Immunol. 2009; 182(12):7663-71,
Molecular Cell, Vol. 7, 867-877, April, 2001,
Nat Biotechnol. 1997 Jul; 15(7):637-40,
Nat Biotechnol. 2005 Oct; 23(10):1283-8,
Proc Natl Acad Sci U S A. 2006 Dec 5; 103(49):18709-14, EP2154157, US20070141052, WO2000/042072, WO2002/060919, WO2006/020114, WO2006/031370, WO2010/033279, WO2006/053301, WO2009/086320.

The Fc region variant to be used in the present invention has FcRn-binding activity. Particularly, it shows high FcRn-binding activity compared to the activity of the Fc region before alteration, and particularly high FcRn-binding activity under acidic conditions.

In the present invention, "having activity" means that, in a system capable of measuring the activity, the measured value becomes higher than the background value (or value when negative control was measured) in the system. For example, having a binding activity means that, in a system capable of measuring the binding activity, such as ELISA, FACS, Biacore and the like, the measured value becomes higher than the background value. In the present invention, the measured value is preferably not less than 2 times, more preferably not less than 3 times, further preferably not less than 5 times, particularly preferably not less than 10 times, higher than the background value.

For example, in the present invention, the reciprocal of KD (dissociation constant) can be used as the value of FcRn binding activity. The KD value of the Fc region variant used in the present invention can be measured by using, for example, a known method of Biacore (GE Healthcare). In the case of Biacore, specifically, the Fc region variant provided by the present invention or an antibody molecule containing the variant is immobilized on a sensor chip, and the KD value can be measured by flowing FcRn as an analyte therein. By performing the measurement in the Fc region of wild-type IgG (wild-type Fc) and the Fc region of mutant IgG (Fc region variant), and under acidic pH conditions and neutral pH conditions, the values of KD (Fc region variant)/KD (wild-type Fc) and KD (pH acidic)/KD (pH neutral) can be calculated.

It is also possible to use kd (Dissociation rate constant) instead of KD.

In the present specification, higher binding activity to dog or cat FcRn than to the parent polypeptide before alteration means that, for example, the activity of binding to dog or cat FcRn is not less than 105%, preferably not less than 110%, not less than 115%, not less than 120%, not less than 125%, particularly preferably not less than 130%, not less than 135%, not less than 140%, not less than 145%, not less than 150%, not less than 155%, not less than 160%, not less than 165%, not less than 170%, not less than 175%, not less than 180%, not less than 185%, not less than 190%, not less than 195%, not less than 2 times, not less than 2.5 times, not less than 3 times, not less than 3.5 times, not less than 4 times, not less than 4.5 times, not less than 5 times, not less than 7.5 times, not less than 10 times, not less than 20 times, not less than 30 times, not less than 40 times, not less than 50 times, not less than 60 times, not less than 70 times, not less than 80 times, not less than 90 times, not less than 100 times, that of the parent polypeptide.

If properties that render the binding activity to dog or cat FcRn stronger than that of natural dog or cat IgG under acidic pH conditions can be imparted to the Fc region variant of the present invention, and if IgG can be constituted using the Fc region variant, the efficiency of recycling from within endosome to within plasma increases since the binding of IgG to FcRn under acidic conditions increases, and as a result, retention in plasma can be improved or enhanced.

In the present invention, the binding activity to dog or cat FcRn under acidic conditions means FcRn binding activity at pH 4.0 - pH 6.5. It preferably means FcRn binding activity at pH 5.0 - pH 6.5, further preferably dog or cat FcRn binding activity at any of pH 5.5, 5.6, 5.7, 5.8, 5.9, 6.0, 6.1, 6.2, 6.3, 6.4, 6.5, particularly preferably, FcRn binding activity at pH 5.8 - pH 6.0, close to the pH in early endosome of living organism. In the present invention, the binding activity to dog or cat FcRn under neutral conditions means FcRn binding activity at pH 6.7 - pH 10.0. Preferably, it means FcRn binding activity at pH 7.0 - pH 9.0, further preferably FcRn binding activity at any of pH 7.0, 7.1, 7.2, 7.3, 7.4, 7.5, 7.6, 7.7, 7.8, 7.9, 8.0, particularly preferably FcRn binding activity at pH 7.4, close to the pH in plasma of living organism.

When it is difficult to measure the binding affinity with FcRn accurately because the affinity is very low at pH 7.4, pH 7.0 can be used instead of pH 7.4. As the temperature used for the measurement conditions, the binding affinity with FcRn may be measured at any temperature of 10°C - 50°C. Preferably, to determine the binding affinity for FcRn, any temperature of 15°C - 40°C is used. Although not particularly limited, 25°C is one of the preferred embodiments.

3. Hinge region

The hinge region is generally located at the C-terminal of the CH1 domain of the heavy chain constant region. In IgG isotypes, disulfide bonds typically occur in this hinge region. In the EPO-Fc fusion protein of the present invention, the EPO polypeptide and the Fc region may be linked via the hinge region or directly linked without via the hinge region. The hinge region may be derived from each immunoglobulin class. The hinge region of cat IgG has three cysteines and the hinge region of dog IgG has three cysteines, at least one of which is involved in the disulfide bond between the heavy chains of immunoglobulin. Accordingly, hinge regions preferred in the present invention are derived from IgG.

In one embodiment, the first cysteine in the hinge region of IgG is preferably substituted with another amino acid, preferably glycine. Another embodiment includes, in addition to the substitution of the first cysteine to glycine, the substitution of a second or third cysteine to glycine, and yet another embodiment includes, in addition to the substitution of the first cysteine to glycine, the substitutions of a second and third cysteine to glycines.

When the Fc region is derived from dog IgG, a hinge region derived from dog IgG is preferably used, and when the Fc region is derived from cat IgG, a hinge region derived from cat IgG is preferably used. As the cat IgG-derived hinge region, those having an amino acid sequence represented by any of SEQ ID NO: 5 - 8 can be mentioned.

When the EPO-Fc fusion protein of the present invention is derived from cat, the sequence of a hinge region to be used is, for example, a sequence shown in any of SEQ ID NO: 5 - 8.

4. Erythropoietin polypeptide (EPO polypeptide)

In the present invention, EPO polypeptide encompasses wild-type or natural erythropoietins, recombinant erythropoietins, as well as erythropoietin-like molecules including biologically active erythropoietin fragments and variants of erythropoietin, derived from any animal species, preferably mammal, more preferably dog or cat, particularly preferably cat.

A dog-derived EPO polypeptide is preferably used when the Fc region is derived from dog IgG, and a cat-derived EPO polypeptide is preferably used when the Fc region is derived from cat IgG. Cat-derived EPO polypeptide includes those having the amino acid sequence shown in SEQ ID NO:9 or 10.

Wild-type or natural erythropoietin is a glycoprotein hormone that stimulates the proliferation and development of erythrocytes from erythropoietin progenitor cells. Wild-type or natural erythropoietin can be routinely isolated and purified from blood or plasma, or from urine.

Erythropoietin synthesized by recombination or chemically can be produced using techniques well known to those of ordinary skill in the art.

In the present specification, the activity (particularly biological activity) of erythropoietin is defined as the ability to stimulate cell proliferation through interactions with erythropoietin receptors. Functional assay of erythropoietin can be performed in vitro or in vivo. For example, the in vitro activity of erythropoietin can be tested in assays using cells. Specifically, erythropoietin activity can be evaluated by a cell proliferation assay using TF-1 cells expressing the EPO receptor. For in vivo activity, for example, hematocrit (HCT) assays and reticulocyte assays using animal models, and the like can be mentioned.

The amino acid sequence of the erythropoietin-like molecule that can be used in the present invention is not particularly limited as long as it has biological activity or functional activity equivalent to that of wild-type or natural erythropoietin. Generally, it has sequence identity of at least about 55%, about 65%, about 75%, typically at least about 80%, about 85%, about 90%, and most typically about 91%, about 92%, about 93%, about 94%, about 95%, about 96%, about 97%, about 98%, about 99%, with the corresponding sequence of wild-type or natural erythropoietin.

The erythropoietin of the present invention is understood to particularly include erythropoietin polypeptides having amino acid sequences similar to those of wild-type erythropoietins. For example, it can contain one or more amino acid alterations in the amino acid sequence of wild-type erythropoietin as long as its biological activity or functional activity is maintained. Examples of such amino acid alteration include addition, deletion or substitution of amino acid residues.

When the EPO-Fc fusion protein of the present invention is derived from cat, the sequence of EPO polypeptide to be used includes the sequence shown in either SEQ ID NO: 9 or 10.

Methods for introducing mutation into erythropoietin are well known in the pertinent technical field. For example, mutations can be introduced by site-directed mutagenesis techniques. A wide range of site-directed mutagenesis techniques can be utilized.

5. Linker

The EPO-Fc fusion protein of the present invention can contain a linker molecule, preferably a peptide linker, between the Fc portion and the erythropoietin portion. Fusion proteins with linkers can have improved properties, such as increased biological activity. Linkers generally contain 1 to 25 amino acids (e.g., 5 to 25 or 10 to 20 amino acids).

6. In vitro activity and in vivo efficacy of EPO-Fc fusion protein

The in vitro activity of EPO-Fc fusion protein can be tested by assays using cells. Particularly, the interaction between the EPO-Fc fusion protein and the EPO receptor (hereinafter to be also abbreviated as EPOR) can be evaluated by TF-1 cell proliferation assay.

The in vivo biological activity of EPO-Fc fusion protein can be measured by assays performed in animal models such as mouse, rat, and the like. Examples of in vivo assay include, but are not limited to, hematocrit (HCT) assay and reticulocyte assay.

7. Synthesis of EPO-Fc fusion protein

In the present invention, a polynucleotide encoding the EPO-Fc fusion protein of the present invention can be provided. Polynucleotide is mainly constituted of DNA, RNA, other nucleic acid analog, and the like. The polynucleotide encoding the EPO-Fc fusion protein of the present invention is constructed and inserted into a suitable expression vector (where necessary, two kinds of expression vectors may also be used). At that time, the gene is incorporated into an expression vector such that it is expressed under control of an expression control region, for example, an enhancer, a promoter. Then, a host cell is transformed with the expression vector and the antibody is expressed. At that time, a suitable combination of a host and an expression vector can be used.

The type of vector that can be used is not particularly limited as long as it stably retains the inserted gene, and various commercially available vectors can be used. Examples of the vector for gene cloning include M13-based vectors, pUC-based vectors, and the like. When a vector is used for the purpose of producing the EPO-Fc fusion protein provided by the present invention, an expression vector is particularly useful. The expression vector is not particularly limited as long as it expresses polypeptide in vitro, in Escherichia coli, in cultured cells, or in an individual organism. Examples of the vector include pBEST vector (manufactured by Promega) and the like as vector for expression in vitro, pGEX, pET, pBluescript vector (manufactured by Stratagene) and the like as vector for expression in Escherichia coli, pME18S-FL3 vector (GenBank Accession No. AB009864) and the like as vector for expression in cultured cells, pcDNA as vector for expression in animal cells, pME18S vector (Mol Cell Biol. 8:466-472(1988)) for expression in individual organisms, and the like. The polynucleotide of the present invention can be inserted into a vector by using, for example, In-Fusion Advantage PCR Cloning Kit (manufactured by Clontech).

The host cell that can be used is not particularly limited and, for example, Escherichia coli, various animal cells, and the like can be preferably used. The host cell can be used, for example, as a production system for producing or expressing the EPO-Fc fusion protein of the present invention. The production system includes in vitro and in vivo production systems. Examples of the in vitro production system include a production system using eukaryotic cells and a production system using prokaryotic cells.

Eukaryotic cell that can be used as a host cell includes, for example, animal cell, plant cell, and fungal cell.

Animal cell includes mammalian cells such as CHO (J. Exp. Med. (1995) 108:94.0), COS, HEK293, 3T3, myeloma, BHK (baby hamster kidney), HeLa, Vero, etc., amphibia cells such as Xenopus oocyte (Valle et al., Nature (1981) 291: 338-340), and insect cells such as Sf9, Sf21, Tn5. Preferably, CHO-DG44, CHO-DX11B, COS7, HEK293, and BHK are used. When a large amount of expression is desired, CHO is particularly preferable. For introduction of a vector into a host cell, for example, a method known to those of ordinary skill in the art such as calcium phosphate method, DEAE dextran method, a method using cationic ribosome DOTAP (manufactured by Boehringer Mannheim), electroporation method, lipofection method, microinjection method and the like can be used. In addition, Free Style 293 Expression System (manufactured by Invitrogen) can also be used to perform steps from gene transfer to polypeptide expression.

The obtained EPO-Fc fusion protein can be isolated intracellularly or extracellularly (medium, milk, and the like) and purified as substantially pure and homogeneous molecules. Separation and purification of the EPO-Fc fusion protein may be performed using the separation and purification method generally used in the purification of polypeptides, and is not limited in any way. For example, column chromatography, filtration, ultrafiltration, salting out, solvent precipitation, solvent extraction, distillation, immunoprecipitation, SDS-polyacrylamide gel electrophoresis, isoelectric focusing, dialysis, recrystallization and the like can be appropriately selected and combined to perform separation and purification.

8. Pharmaceutical composition

The present invention also provides a pharmaceutical composition containing EPO-Fc fusion protein produced according to the present invention. While pharmaceutical compositions can be used for treating diseases, for example, the pharmaceutical composition provided by the present invention can be used for stimulating erythrocyte formation, and prevent or treat anemia. In the present specification, "treatment" means to obtain pharmacological and/or physiological effects. The effect can be prophylactic in that it completely or partially prevents the symptoms of the disease, and can also be therapeutic in that it completely or partially treats the symptoms of the disease. The "treatment" in the present specification includes all treatments for diseases in mammals, particularly dogs or cats, or animal species closely related thereto.

The pharmaceutical composition provided by the present invention can be formulated by a method known to those of ordinary skill in the art (e.g., Remington's Pharmaceutical Science, latest edition, Mark Publishing Company, Easton, USA). Generally, it contains pharmaceutically acceptable additives that are conventionally used in the art and suitable for administration to a subject for therapeutic, diagnostic or prophylactic purposes. For example, when formulated as a solid, for example, a filler such as lactose and the like, a binder such as carboxymethyl cellulose, gelatin and the like, a coloring agent, a coating agent and the like can be used, and such agent is suitable for oral administration. In addition, for example, white petrolatum, a cellulose derivative, a surfactant, polyethylene glycol, silicone, olive oil, and the like may be added as a carrier or an excipient and applied to the affected part as an external medicine in the form of cream, milky lotion, lotion or the like. When formulated as a liquid, it can contain generally-used physiologically acceptable solvent, emulsifier, and stabilizer. Examples of the solvent include water, PBS, isotonic physiological saline and the like; examples of the emulsifier include polyoxyethylene-based surfactant, fatty acid-based surfactant, silicone, and the like; and examples of the stabilizer include dog serum albumin, polyols such as gelatin and the like, saccharides such as sorbitol, trehalose and the like, and the like. Composition for oral administration can form solution, suspension, tablet, pill, capsule, sustained release formulation, mouthwash or powder.

While the method for administering the pharmaceutical composition of the present invention is not particularly limited, therapeutic effects can be expected most by injection administration. The injection administration method is not limited to any of intravenous administration, intramuscular administration, subcutaneous administration, intraperitoneal administration, and intrathoracic administration.

The dose is determined depending on the type of EPO-Fc fusion protein used, size of individual, administration method, type of disease, symptoms, and the like. It only needs to be administered in an amount sufficient to show a therapeutic effect and a prophylactic effect.

All prior art documents cited in the present specification are incorporated in the present specification for reference.

[Example]

The present invention is now explained further in the following by referring to Reference Examples, Experimental Examples, and Examples, which are not to be construed as limitative.

Reference Example 1. Preparation of IgG expression vector having wild-type Fc

Gene synthesis of the Fc region of wild-type dog IgG H chain registered in GenBank:AF354265.1 (SEQ ID NO: 1, hereinafter dog wild type Fc, also abbreviated as dog wtFc) and the Fc region of wild-type cat IgG H chain registered in GenBank:AB016710.1 (SEQ ID NO: 2, hereinafter cat wild type Fc, also abbreviated as cat wtFc) was performed by GenScript Japan Inc. based on the amino acid sequence.

Gene synthesis of the region from Fd to the hinge of the IgG H chain (SEQ ID NO: 18, hereinafter Fd-Hinge) and the entire region of the IgG L chain (SEQ ID NO: 19, hereinafter L chain) was performed by GenScript Japan Inc. based on the amino acid sequence of a humanized anti-human IgE antibody omalizumab registered in IMGT (reference URL http://www.imgt.org/) under IMGT/mAb-DB ID:77, such that an amino acid consisting of MEFGLSWVFLVALFRGVQC (SEQ ID NO: 20) is attached as a secretory signal peptide to the N-terminal side of the Fd-Hinge and an amino acid consisting of MDMRVPAQLLGLLLLWLSGARC (SEQ ID NO: 21) is attached to the N-terminal side of the L chain.

The synthesized Fd-Hinge gene of omalizumab containing the secretory signal peptide was amplified by the PCR method, connected using the In-Fusion HD Cloning Kit (manufactured by Clontech) (hereinafter In-Fusion Kit) to each of dog wtFc gene and cat wtFc gene similarly amplified by the PCR method such that Fd-Hinge was on the N-terminal side and wtFc on the C-terminal side, simultaneously inserted directly under the CMV promoter of pcDNA3.1(+) (Invitrogen), Escherichia coli DH5α was transformed, and the plasmid was extracted to give H chain expression vectors pcDNA3.1(+)/omalizumab Fd-dog wtFc and pcDNA3.1(+)/omalizumab Fd-cat wtFc.

The synthesized L chain gene of omalizumab containing the secretory signal peptide was amplified by the PCR method, inserted directly under the CMV promoter of pcDNA3.1(+) (Invitrogen) using the In-Fusion kit, Escherichia coli DH5α was transformed, and the plasmid was extracted to give L chain expression vector pcDNA3.1(+)/omalizumab Lch.

In all cases, when using the In-Fusion kit, Escherichia coli DH5α competent cells (TOYOBO) were transformed with the DNA solution after the In-Fusion reaction, according to the method described in the attached manual. The obtained transformant was cultured overnight at 37°C in LB liquid medium containing 100 ug/mL ampicillin, and a plasmid was extracted therefrom using the NucleoBond Xtra Midi Kit (Takara Bio Inc.). The base sequence of the obtained expression vector was determined by a method known to those of ordinary skill in the art, and it was confirmed that the protein of the amino acid sequence of interest was encoded.

amino acid sequence of omalizumab Fd-Hinge region

```
EVQLVESGGGLVQPGGSLRLSCAVSGYSITSGYSWNWIRQAPGKGLEWVASITYDGSTNYNPS
VKGRITISRDDSKNTFYLQMNSLRAEDTAVYYCARGSHYFGHWHFAVWGQGTLVTVSSASTKG
PSVFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCP(SEQ ID NO: 18)
```

amino acid sequence of wild-type dog IgG H chain Fc region

```
APEMLGGPSVFIFPPKPKDTLLIARTPEVTCVVVDLDPEDPEVQISWFVDGKQMQTAKTQPRE
EQFNGTYRVVSVLPIGHQDWLKGKQFTCKVNNKALPSPIERTISKARGQAHQPSVYVLPPSRE
ELSKNTVSLTCLIKDFFPPDIDVEWQSNGQQEPESKYRTTPPQLDEDGSYFLYSKLSVDKSRW
QRGDTFICAVMHEALHNHYTQESLSHSPGK(SEQ ID NO: 1)
```

amino acid sequence of wild-type cat IgG H chain Fc region

```
PPEMLGGPSIFIFPPKPKDTLSISRTPEVTCLVVDLGPDDSDVQITWFVDNTQVYTAKTSPRE
EQFNSTYRVVSVLPILHQDWLKGKEFKCKVNSKSLPSPIERTISKAKGQPHEPQVYVLPPAQE
ELSRNKVSVTCLIKSFHPPDIAVEWEITGQPEPENNYRTTPPQLDSDGTYFVYSKLSVDRSHW
QRGNTYTCSVSHEALHSHHTQKSLTQSPGK(SEQ ID NO: 2)
```

amino acid sequence of omalizumab L chain

DIQLTQSPSSLSASVGDRVTITCRASQSVDYDGDSYMNWYQQKPGKAPKLLIYAASYLESGVP

SRFSGSGSGTDFTLTISSLQPEDFATYYCQQSHEDPYTFGQGTKVEIKRTVAAPSVFIFPPSD

EQLKSGTASVVCLLNNFYPREAKVQWKVDNALQSGNSQESVTEQDSKDSTYSLSSTLTLSKAD

YEKHKVYACEVTHQGLSSPVTKSFNRGEC(SEQ ID NO: 19)

Reference Example 2. Preparation of IgG H chain expression vector having altered Fc

Primers encoding mutant amino acids were designed so that the amino acid sequence of the Fc region would be substituted by the amino acid shown in Table 1 *"Position of dog, cat Fc alteration"*. Using H chain expression vectors pcDNA3.1(+)/omalizumab Fd-dog wtFc and pcDNA3.1(+)/omalizumab Fd-cat wtFc prepared in Example 1 as templates, a DNA fragment in which a mutation was introduced into the Fc region was amplified by the PCR method using the designed primers, and an H-chain expression vector in which a mutation was introduced only into arbitrary sites of the Fc serving as the template was prepared by ligating the amplified DNA fragments by using the In-Fusion kit.

In all cases, when using the In-Fusion kit, Escherichia coli DH5α competent cells (TOYOBO) were transformed with the DNA solution after the In-Fusion reaction, according to the method described in the attached manual. The obtained transformant was cultured overnight at 37°C in LB liquid medium containing 100 ug/mL ampicillin, and a plasmid was extracted therefrom using the NucleoBond Xtra Midi Kit (Takara Bio Inc.). The base sequence of the obtained expression vector was determined by a method known to those of ordinary skill in the art, and it was confirmed that the protein of the amino acid sequence of interest was encoded.

Reference Example 3. Antibody expression and purification

The expression vectors obtained in Reference Example 1 and Reference Example 2 were transiently introduced into FreeStyle 293 cells (Invitrogen) to express the antibody. After collecting the obtained culture supernatant, the culture supernatant was obtained by passing through a 0.22 um filter Millex(R)-GP (Merck Millipore). From the obtained culture supernatant, the antibody was purified by affinity chromatography using MabSelect SuRe (GE Healthcare), elution with 50 mM acetic acid, and neutralization treatment by the addition of 1.5M Tris-HCl, pH 7.5. The obtained antibody was subjected to buffer substitution with a buffer of 20 mM Histidine-HCl, 150 mM NaCl, pH 6.5, using an ultrafiltration membrane (Merck Millipore) capable of fractionating 30 kDa. For the purified antibody concentration, the absorption at 280 nm was measured using a spectrophotometer, and the antibody concentration was calculated from the obtained value and using the absorption coefficient calculated by the method of PACE et al. (Protein Science (1995); 4, 2411-2423).

Reference Example 4. Preparation of FcRn Expression Vector

Gene synthesis of an extracellular region of dog FCGRT registered in GenBank: XP_005616366.1 (SEQ ID NO: 22, hereinafter also abbreviated as dog FCGRT) and extracellular region of cat FCGRT registered in GenBank: XP_023100998.1 (SEQ ID NO: 23, hereinafter also abbreviated as cat FCGRT) to afford each amino acid sequence with Histag (HHHHHHHH) (SEQ ID NO: 24) attached to the C-terminal side, and cloning of the synthesized genes into pcDNA3.1(+) (Invitrogen) and the plasmid extraction were performed by GenScript Japan Inc. The obtained expression vectors were pcDNA3.1(+)/dog FCGRT, pcDNA3.1(+)/cat FCGRT.

Gene synthesis of dogβ2m registered in GenBank: NP_001271408 (SEQ ID NO: 25, hereinafter also abbreviated as dogβ2m) and catβ2m registered in GenBank: NP_001009876 (SEQ ID NO: 26, hereinafter also abbreviated as catβ2m) based on the amino acid sequence, and cloning of the synthesized genes into pcDNA3.1(+) (Invitrogen) and the plasmid extraction were performed by GenScript Japan Inc. The obtained expression vectors were pcDNA3.1(+)/dogβ2m, pcDNA3.1(+)/catβ2m.

amino acid sequence of dog FCGRT extracellular region

MGVPRPRSWGLGFLLFLLPTLRAADSHLSLLYHLTAVSAPPPGTPAFWASGWLGPQQYLSYNN
LRAQAEPYGAWVWENQVSWYWEKETTDLRTKEGLFLEALKALGDGGPYTLQGLLGCELGPDNT
SVPVAKFALNGEDFMTFDPKLGTWNGDWPETETVSKRWMQQAGAVSKERTFLLYSCPQRLLGH
LERGRGNLEWKEPPSMRLKARPGSPGFSVLTCSAFSFYPPELQLRFLRNGLAAGSGEGDFGPN
GDGSFHAWSSLTVKSGDEHHYRCLVQHAGLPQPLTVELESPAKSS(SEQ ID NO: 22)

amino acid sequence of cat FCGRT extracellular region

MGVPRPQPWGLGFLLFLLPTLRAAESHLSLLYHLTAVSSPAPGTPAFWVSGWLGPQQYLSYNN
LRAQAEPCGAWVWENQVSWYWEKETTDLRNKQELFLEALKVLGEGGPYTLQGLLGCELGPDNA
SVPVAKFALNGEDFMDFDPKLGTWSGEWPETETISKRWMQEAGAVSKERTFLLNSCPQRLLGH
LERGRGNLEWKEPPSMRLKARPGSPGFSVLTCSAFSFYPPELQLRFLRNGLAAGSGEGDFGPN
GDGSFHAWSSLTVKSGDEHHYRCLVQHAGLPQPLTVELESPAKSS(SEQ ID NO: 23)

amino acid sequence of dog β2m

MAPRPALATAGFLALLLILLAACRLDAVQHPPKIQVYSRHPAENGKPNFLNCYVSGFHPPEIE
IDLLKNGKEMKAEQTDLSFSKDWTFYLLVHTEFTPNEQDEFSCRVKHVTLSEPQIVKWDRDN(
SEQ ID NO: 25)

amino acid sequence of cat β2m

MARFVVLVLLGLLYLSHLDAVQHSPKVQVYSRHPAENGKPNFLNCYVSGFHPPQIDITLMKNG
KKMEAEQTDLSFNRDWTFYLLVHTEFTPTVEDEYSCQVNHTTLSEPKVVKWDRDM(SEQ ID

NO: 26)

Reference Example 5. Expression and Purification of FcRn Protein

The expression vectors in a combination of pcDNA3.1(+)/dog FCGRT and pcDNA3.1(+)/dogβ2m, and a combination of pcDNA3.1(+)/cat FCGRT and pcDNA3.1(+)/catβ2m obtained in Example 4 were co-transfected into FreeStyle 293 cells (Invitrogen) to express dog and cat FcRn proteins. After culturing and collecting the obtained culture supernatant, the culture supernatant was obtained by passing through a 0.22 um filter Millex(R)-GP (Merck Millipore). The obtained culture supernatant was purified in the following two steps in principle. In the first step, affinity column chromatography on His tags (His Trap HP) was performed and the protein of interest was fractionated by gradient elution of imidazole concentration using buffers of 20 mM Tris, 0.5 M NaCl, 10 mM imidazole, pH 7.4 and 20 mM Tris, 0.5 M NaCl, 500 mM imidazole, pH 7.4. In the second step, substitution with D-PBS(-), pH 7.0 buffer and size fractionation were performed using gel filtration column chromatography (Superdex200) to purify the protein of interest. For the purified protein, the absorption at 280 nm was measured using a spectrophotometer, and the concentration of the purified protein was calculated from the obtained value and using the absorption coefficient calculated by the method of PACE et al. (Protein Science (1995); 4, 2411-2423) .

Experimental Example 1: Interaction measurement by Biacore (binding analysis)

Evaluation of binding ability of obtained antibody to dog and cat FcRns

The obtained antibody was evaluated using BiacoreX100 (GE Healthcare) to determine whether it has the binding ability to dog and cat FcRn. As the condition in plasma, pH 7.4 was set. As the condition in endosome (acidic condition), pH 6.0 was set. The antibody of interest was captured by Sensor chip Protein L (GE Healthcare), and dog and cat FcRns were used as antigens. The measurement was performed using three kinds of running buffers (1; 50 mmol/L phosphoric acid, 150 mmol/L NaCl, 0.05%(w/v) Tween-20, pH 7.4, 2; 50 mmol/L phosphoric acid, 150 mmol/L NaCl, 0.05%(w/v) Tween-20, pH 7.0, 3; 50 mmol/L phosphoric acid, 150 mmol/L NaCl, 0.05%(w/v) Tween-20, pH 6.0).

Method for performing measurement

The antibody diluted with a running buffer was injected at a flow rate of 5 $\mu$L/min for 1 min to allow for capture by a sensor chip. Then, FcRn diluted to 1600, 800, 400, 200, 100 nM with the running buffer and the running buffer (as a reference solution) were injected at a flow rate of 30 $\mu$L/min for 2 min to cause interaction with the captured antibody. Furthermore, the running buffer was flown for 10 min at a flow rate of 30 $\mu$L/min to observe dissociation of FcRn. Finally, 10 mmol/L Glycine-HCl, pH 1.7 was injected twice at a flow rate of 30 $\mu$L/min for 1 min to regenerate the sensor chip. The antibody captured on the sensor chip was washed by the regeneration operation, and the sensor chip was used repeatedly.
Since the binding affinity between wild-type IgG and FcRn is very low at pH 7.4, calculation of the KD value is difficult. Therefore, when accurate measurement of the affinity is difficult, the measurement was performed using pH 7.0 instead of pH 7.4.

Analysis method

To calculate the dissociation constant KD (mol/L) to FcRn of the antibody containing each Fc region variant, kinetic analysis was performed according to the following method. First, the desired antibody was captured using the above-mentioned sensor chip, allowed to interact with FcRn diluted with running buffer. The measurement results were globally fitted to the obtained sensorgram by Biacore Evaluation Software in a 1:1 binding model to calculate the binding rate constant ka (L/mol/s) and the dissociation rate constant kd (1/s), and the dissociation constant KD (mol/L) was calculated from the values.
When the obtained sensorgram is box-shaped and reaches an equilibrium state immediately, the equilibrium value (= binding amount) during FcRn injection reflects the dissociation constant KD(M). The dissociation constant KD (mol/L) of each variant to FcRn was calculated by performing steady state affinity analysis using Biacore Evaluation Software on the sensorgram obtained as the measurement result of Biacore.
The behavior of molecules interacting in the 1:1 binding model on Biacore can be expressed by the formula 1 below.

$$\text{Req} = \text{C} \times \text{Rmax}/(\text{KD} + \text{C}) + \text{RI} \quad \text{(formula 1)}$$

Each item in the above-mentioned formula means as follows.

Req(RU): steady state binding levels
Rmax(RU): affinity binding capacity of the surface of analyte
RI(RU): bulk refractive index contribution in the sample
C(M): analyte concentration
KD(M): equilibrium dissociation constant

The results are shown in the following Tables.

EP 4 166 574 A1

[Table 2]

Comparison of KD (mol/L) values of dog Fc region variant and dog FcRn

| name of variant | KD (mol/L) under measurement pH condition | | | | | | KD (variant)/ KD (wild-type) at pH 6.0 | KD(pH6.0) /KD (pH7.4) | KD(pH6.0) /KD (pH7.0) |
| --- | --- | --- | --- | --- | --- | --- | --- | --- | --- |
| | pH7.4 | | pH7.0 | pH6.0 | | | | | |
| Wild-type Fc | | | 2.37E-06 | 1.99E-06 | 1.77E-06 | 2.01E-06 | 1.00 | | 0.840 |
| DFV-1 | | | | 5.43E-07 | | | 0.27 | | |
| DFV-2 | 3.43E-06 | | | 3.87E-07 | | | 0.19 | 0.113 | |
| DFV-3 | | | | 3.71E-07 | | | 0.19 | | |
| DFV-4 | 1.81E-05 | | | 1.09E-07 | | | 0.05 | 0.006 | |
| DFV-5 | 2.30E-06 | | | | 8.20E-07 | | 0.46 | 0.357 | |
| DFV-6 | | 2.64E-07 | | | | 2.63E-08 | 0.01 | 0.100 | |
| DFV-7 | | | | | | 2.03E-06 | 1.01 | | |
| DFV-8 | | 6.85E-06 | | | | 6.96E-08 | 0.03 | 0.010 | |

[Table 3]

Compar cat Fc ison of Rn KD ( mol/L) values of cat Fc reg ion varia nt and

| name of variant | KD (mol /L) under con measurem dition ent pH | | | | KD (variant)/ at KD (wild-type) pH 6.0 | KD (pH6.0) /KD (pH7.4) | KD (pH6.0) /KD (pH7.0) |
| --- | --- | --- | --- | --- | --- | --- | --- |
| | pH7.4 | pH7.0 | pH 6.0 | | | | |
| Wild-type Fc | | 7.58E-06 | 6.32E-07 | 7.00E-07 | 1.00 | | 0.083 |
| CFV-1 | | | 4.04E-07 | | 0.64 | | |
| CFV-2 | 1.41E-05 | | 6.48E-08 | | 0.10 | 0.005 | |
| CFV-3 | | | 4.62E-08 | | 0.07 | | |
| CFV-4 | 4.61E-06 | | 2.14E-08 | | 0.03 | 0.005 | |
| CFV-6 | 1.06E-06 | | | 2.70E-09 | 0.00 | 0.003 | |
| CFV-7 | | | | 5.10E-08 | 0.07 | | |
| CFV-8 | 6.39E-06 | | | 3.93E-08 | 0.06 | 0.006 | |

18

[Table 4]

| Comparison of KD (mol/L) values of dog Fc region variant and dog FcRn at pH 6.0 | | | | |
|---|---|---|---|---|
| name of variant | KD (mol/L) under pH 6.0 condition | | | activity of Fc region variant with FcRn binding activity of wild-type Fc as 1 |
| wild-type Fc | 1.99E-06 | 1.77E-06 | 2.01E-06 | 1.0 |
| DFV-1 | 5.43E-07 | | | 3.7 |
| DFV-2 | 3.87E-07 | | | 5.1 |
| DFV-3 | 3.71E-07 | | | 5.4 |
| DFV-4 | 1.09E-07 | | | 18.2 |
| DFV-5 | | 8.20E-07 | | 2.2 |
| DFV-6 | | | 2.63E-08 | 76.3 |
| DFV-7 | | | 2.03E-06 | 1.0 |
| DFV-8 | | | 6.96E-08 | 28.8 |

[Table 5]

| Comparison of KD (mol/L) values of cat Fc region variant and cat FcRn at pH 6.0 | | | |
|---|---|---|---|
| name of variant | KD (mol/L) under pH 6.0 condition | | activity of Fc region variant with FcRn binding activity of wild-type Fc as 1 |
| wild-type Fc | 6.32E-07 | 7.00E-07 | 1.0 |
| CFV-1 | 4. 04E-07 | | 1.6 |
| CFV-2 | 6.48E-08 | | 9.8 |
| CFV-3 | 4.62E-08 | | 13.7 |
| CFV-4 | 2.14E-08 | | 29.5 |
| CFV-6 | | 2.70E-09 | 259.3 |
| CFV-7 | | 5.10E-08 | 13.7 |
| CFV-8 | | 3.93E-08 | 17.8 |

Example 1. Construction of expression plasmid for fusion protein of cat wild-type erythropoietin and IgG H chain Fc region

An expression plasmid for a fusion protein of cat erythropoietin (hereinafter to be referred to as EPO) and IgG H chain Fc region (hereinafter to be referred to as Fc) (hereinafter to be referred to as cat EPO-Fc or cat EPO-Fc) was constructed. The detail is shown below.

By reference to the amino acid sequence (SEQ ID NO: 11) of the wild-type cat EPO registered in GenBank: JQ413414.1, and the amino acid sequence from the hinge region (SEQ ID NO: 12) to the Fc region (SEQ ID NO: 2) of the wild-type cat IgG H chain registered in GenBank: AB016710.1, the amino acid sequence of cat EPO-Fc was determined (SEQ ID NO: 13). At this time, the EPO region and Fc region were left as wild-type sequences, and in the hinge region, of the three cysteines, one present on the N-terminal side was altered to glycine. A gene was synthesized based on the determined amino acid sequence.

Plasmid pCAG (structure shown in Fig. 1) constructed in-house was cleaved with restriction enzymes SbfI and EcoRV (both New England Biolabs) to prepare a plasmid fragment opened directly under the CAG promoter, and ligated to a gene fragment of cat EPO-Fc synthesized using In-Fusion HD Cloning kit (manufactured by Clontech) (hereinafter referred to as In-Fusion kit) to construct expression plasmid pCAG/C2 cat wtEPO-Fc for wild-type cat EPO-Fc. For use of the In-Fusion kit, Escherichia coli DH5α competent cells (TOYOBO) were transformed with the DNA solution after the In-Fusion reaction, according to the method described in the attached manual. The obtained transformant was cultured overnight at 37°C in LB liquid medium containing 25 ug/mL kanamycin, and a plasmid was extracted using the NucleoBond Xtra Midi Kit (Takara Bio Inc.) from Escherichia coli obtained by cultivation. The base sequence of the obtained plasmid was determined by a method known to those skilled in the art, and it was confirmed that the protein of the amino acid sequence of interest was encoded.

wild-type cat EPO

```
MGSCECPALLLLLSLLLLPLGLPVLGAPPRLICDSRVLERYILEAREAENVTMGCAEGCSFSE
NITVPDTKVNFYTWKRMDVGQQAVEVWQGLALLSEAILRGQALLANSSQPSETLQLHVDKAVS
SLRSLTSLLRALGAQKEATSLPEATSAAPLRTFTVDTLCKLFRIYSNFLRGKLTLYTGEACRR
GDR(SEQ ID NO: 11)
```

hinge region of wild-type cat IgG H chain
RKTDHPPGPKPCDCPKCP(SEQ ID NO: 12)

Fc region of wild-type cat IgG H chain

```
PPEMLGGPSIFIFPPKPKDTLSISRTPEVTCLVVDLGPDDSDVQITWFVDNTQVYTAKTSPRE
EQFNSTYRVVSVLPILHQDWLKGKEFKCKVNSKSLPSPIERTISKAKGQPHEPQVYVLPPAQE
ELSRNKVSVTCLIKSFHPPDIAVEWEITGQPEPENNYRTTPPQLDSDGTYFVYSKLSVDRSHW
QRGNTYTCSVSHEALHSHHTQKSLTQSPGK(SEQ ID NO: 2)
```

genetically-synthesized cat EPO-Fc

```
MGSCECPALLLLLSLLLLPLGLPVLGAPPRLICDSRVLERYILEAREAENVTMGCAEGCSFSE
NITVPDTKVNFYTWKRMDVGQQAVEVWQGLALLSEAILRGQALLANSSQPSETLQLHVDKAVS
SLRSLTSLLRALGAQKEATSLPEATSAAPLRTFTVDTLCKLFRIYSNFLRGKLTLYTGEACRR
GDRRKTDHPPGPKPGDCPKCPPPEMLGGPSIFIFPPKPKDTLSISRTPEVTCLVVDLGPDDSD
VQITWFVDNTQVYTAKTSPREEQFNSTYRVVSVLPILHQDWLKGKEFKCKVNSKSLPSPIERT
ISKAKGQPHEPQVYVLPPAQEELSRNKVSVTCLIKSFHPPDIAVEWEITGQPEPENNYRTTPP
QLDSDGTYFVYSKLSVDRSHWQRGNTYTCSVSHEALHSHHTQKSLTQSPGK(SEQ ID NO:
13)
```

Example 2. Construction of expression plasmid for cat EPO-Fc with altered amino acid in EPO region, hinge region

In order to search for amino acid mutations that change the properties and physiological activity of wild-type cat EPO-Fc, expression plasmids for amino acid mutants of cat EPO-Fc (hereinafter also referred to as cat EPO-Fc variants) were constructed, in which cysteine in the EPO region, cysteine in the hinge region, and the presence or absence of the hinge region were variously changed as shown in Table 6. The detail is shown below.

First, pCAG/C2 cat wtEPO-Fc constructed in Example 1 was annealed to the mutation introduction sites of cat EPO-Fc DNA, and primers encoding the amino acid sequences characteristic of respective cat EPO-Fc variants shown in Table 6 were designed. Then, using pCAG/C2 cat wtEPO-Fc as templates, DNA fragments in which mutations were introduced were amplified by the PCR method using the designed primers, and the amplified DNA fragments were ligated using the In-Fusion kit to construct expression plasmids pCAG/C2 C59P cat EPO-Fc, pCAG/C2 C165R cat EPO-Fc, pCAG/C1a cat wtEPO-Fc, pCAG/C1a C59P cat EPO-Fc, pCAG/C1a C165R cat EPO-Fc, pCAG/C1b cat wtEPO-Fc, pCAG/C1b C59P cat EPO-Fc, pCAG/C1b C165R cat EPO-Fc, pCAG/C0 cat wtEPO-Fc, pCAG/C0 C59P cat EPO-Fc, pCAG/C0 C165R cat EPO-Fc, pCAG/HL cat wtEPO-Fc, pCAG/HL C59P cat EPO-Fc, pCAG/HL C165R cat EPO-Fc for cat EPO-Fc variant. Use of the In-Fusion kit, and subsequent transformation and culture of Escherichia coli DH5α, extraction of plasmid, and determination of base sequence were performed in the same manner as the method described in Example 1, and construction of the plasmid of interest was confirmed.

[Table 6]

| cat EPO-Fc with altered amino acids in EPO region and hinge region | | | |
|---|---|---|---|
| plasmid | EPO sequence | hinge sequence | Fc sequence |
| pCAG/C2 cat wtEPO-Fc | wild type | C2:RKTDHPPGPKPGDCPKCP (SEQ ID: No 5) | wild type |
| pCAG/C2 C59P cat EPO-Fc | C59P ∗ | | |
| pCAG/C2 C165R cat EPO-Fc | C165R ∗∗ | | |
| pCAG/C1 a cat wtEPO-Fc | wild type | C1a:RKTDHPPGPKPGDGPKCP (SEQ ID: No 6) | |
| pCAG/C1 a C59P cat EPO-Fc | C59P ∗ | | |
| pCAG/C1 a C165R cat EPO-Fc | C165R ∗∗ | | |
| pCAG/C1 b cat wtEPO-Fc | wild type | C1b:RKTDHPPGPKPGDCPKGP (SEQ ID: No 7) | |
| pCAG/C1 b C59P cat EPO-Fc | C59P ∗ | | |
| pCAG/C1 b C165R cat EPO-Fc | O165R ∗∗ | | |
| pCAG/C0 cat wtEPO-Fc | wild type | CO:RKTDHPPGPKPGDGPKGP (SEQ ID: No 8) | |
| pCAG/C0 C59P cat EPO-Fc | C59P ∗ | | |
| pCAG/C0 C165R cat EPO-Fc | C16SR ∗∗ | | |
| pCAG/HL cat wtEPO-Fc | wild type | HL: - | |
| pCAG/HL C59P cat EPO-Fc | C59P ∗ | | |
| pCAG/HL 0165 R cat EPO-Fc | C1 65R ∗∗ | | |
| *C59P means that the 59th amino acid from the start Met of wild-type cat EPO was changed from Cys to Pro. **C165R means that the 165th amino acid from the start Met of wild-type cat EPO was changed from Cys to Arg. | | | |

Refer to SEQ ID NO: 11 for the sequence of wild-type EPO. Refer to SEQ ID NO: 2 for the sequence of wild-type Fc.

Example 3. Expression, purification, and analysis of wild-type cat EPO-Fc-, EPO region-, and hinge region-altered cat EPO-Fc protein

Using the ExpiCHO™ Expression System Kit (Gibco), which includes cells, medium, and transfection reagent and according to the Max Titer protocol of the kit, each of the 15 kinds of cat EPO-Fc expression plasmids obtained in Example 1 and Example 2 was introduced into Expi CHO-S™ cells and cultured for 14 days to allow for protein expression. The culture supernatant (30 mL) after the transfection was clarified through a 0.22 μm filter Millex(R)-GP (Merck Millipore).
A portion of the clarified culture supernatant was electrophoresed by SDS-PAGE under non-reducing conditions using Novex™ 4-20% Tris-Glycine Mini gel (Invitrogen), transferred to a PVDF membrane (Invitrogen), and the protein of interest contained in the culture supernatant was analyzed by Western Blot method using Rabbit anti-Human Erythropoietin Antibody (R&D Systems) as the primary antibody, Goat Anti-Rabbit IgG Antibody (AP) (Millipore) as the secondary antibody, and BCIP-NBT Solution Kit for Alkaline Phosphatase Stain, Nuclease tested (Nacalai Tesque) as a color development substrate (Fig. 2).
In SDS-PAGE under non-reducing conditions, cat EPO-Fc variants with C2 type, C1a type, or C1b type hinge showed the main bands at about twice the size (between 130-250 kDa) of the main band (55-70 kDa) of cat EPO-Fc variants with C0 type or HL type hinge. Therefrom it was shown that cat EPO-Fc variants having C2 type, C1a type, or C1b type hinge have intermolecular disulfide bonds. Furthermore, since the cat EPO-Fc variant with C165R mutation in the EPO region had a very faint smear band compared to other variants, it was shown that the variant contained less associations and aggregates other than dimers and that it had superior properties.

The remaining total volume of the clarified culture supernatant was added to a column filled with MabSelect SuRe (GE Healthcare Lifesciences), and protein A affinity purification of expressed proteins was performed using 50 mM acetic acid as an elution buffer, and 1.5 M Tris-HCl, pH 7.5, as a neutralization buffer. Thereafter, gel filtration chromatography using a Superdex 200 gel filtration column (GE Healthcare Lifesciences) and 50 mM phosphoric acid and 300 mM NaCl, pH 7.0, as buffers was further performed, and a protein fraction of the main peak was separated to obtain a purified protein. Using a spectrophotometer, the absorbance of the purified protein at 280 nm was measured, and the yield of the purified protein was calculated using the absorption coefficient calculated from the obtained value by the method of PACE et al. (Protein Science (1995); 4, 2411-2423) (Table 7).

[Table 7]

|  |  | mg |
|---|---|---|
| 1 | wt catEPO-C2-Fc | 0.314 |
| 2 | C59P catEPO-C2-Fc | 0.323 |
| 3 | C165R catEPO-C2-Fc | 0.453 |
| 4 | wt catEPO-C1 a-Fc | 0.257 |
| 5 | C59P catEPO-C1 a-Fc | 0.299 |
| 6 | C165R catEPO-C1 a-Fc | 0.45 |
| 7 | wt catEPO-C1 b-Fc | 0.372 |
| 8 | C59P catEPO-C1 b-Fc | 0.354 |
| 9 | C165R catEPO-C1 b-Fc | 0.275 |
| 10 | wt catEPO-CO-Fc | 0.349 |
| 11 | C59P catEPO-C0-Fc | 0.371 |
| 12 | C1 65R catEPO-C0-Fc | 0.397 |
| 13 | wt catEPO-HL-Fc | 0.486 |
| 14 | C59P catEPO-HL-Fc | 0.625 |
| 15 | C1 65R catEPO-HL-Fc | 0.671 |

It was found that the protein yield after purification increases in cat EPO-Fc variants with C2 type, C1a type, or HL type hinge when the C165R mutation is added to the EPO region in combination with the hinge mutation. When the protein was purified by gel filtration, only the main peak was fractionated as much as possible, and contaminant peaks assumed to be protein aggregates and degradation products were not fractionated. Therefore, the good yield of cat EPO-Fc having EPO region C165R, and C2 type, C1a type, or HL type hinges was considered to be attributable to superior properties.

The cat EPO-Fc after purification was subjected to SDS-PAGE under non-reducing and reducing conditions using Novex™ 4-20% Tris-Glycine Mini gel (Invitrogen) and CBB staining with SimplyBlue™ SafeStain (Invitrogen), and the presence or absence of an intermolecular disulfide bond was confirmed in cat EPO-Fc (Fig. 3).

The main band of cat EPO-Fc variant with C2 type, C1a type, or C1b type hinge is positioned between 55 and 70 kDa under reducing conditions, and at about twice the size (between 130-250 kDa) under non-reducing conditions. Therefrom it was shown that cat EPO-Fc variants having C2 type, C1a type, or C1b type hinge form dimers having intermolecular disulfide bonds. In addition, it was shown that since the main band of cat EPO-Fc variants with C0 type or HL type hinge is always located between 55 and 70 kDa regardless of non-reducing or reducing, at least a covalent dimer containing an intermolecular disulfide bond is not formed. On the other hand, since cat EPO-Fc variants with C0-type or HL-type hinge still have Fc, it is considered that they form non-covalent dimers mediated by Fc.

Furthermore, cat EPO-Fc purified by size-exclusion chromatography (SEC) apparatus using G3000SWXL column (Tosoh Corporation) as a carrier, 50 mM phosphoric acid and 300 mM NaCl, pH 7.0, as a mobile phase, flow rate of 0.7 mL/min, and detection wavelength of 220 nm was C-analyzed. The peak eluted earlier than the main peak (dimer) was analyzed as aggregates and associations, the peak eluted later was analyzed as monomers and degraded products, the content (%) of the main peak was calculated by the area percentage method, and compared between samples together with the retention time (RT) (Table 8) (Fig. 4).

[Table 8]

| | | | RT(min) | area(%) | | | | RT(min) | area (%) |
|---|---|---|---|---|---|---|---|---|---|
| retention time (RT) and content (%) of main peak in SEC analysis of EPO region- and hinge region-altered cat EPO-Fc | | | | | | | | | |
| 1 | wt catEPO-C2-Fc | | 10.949 | 98.35 | | 10 | wt catEPO-C0-Fc | 10.723 | 98.19 |
| 2 | C59P catEPO-C2-Fc | | 10.952 | 99.53 | | 11 | O59P catEPO-C0-Fc | 10.714 | 99.36 |
| 3 | C165R catEPO-C2-Fc | | 10.959 | 99.42 | | 12 | C165R catEPO-C0-Fc | 10.727 | 98.95 |
| 4 | wt catEPO-C1 a-Fc | | 10.92 | 98.08 | | 13 | wt catEPO-HL-Fc | 11.062 | 98.47 |
| 5 | O59P catEPO-C1a-Fc | | 10.936 | 99.23 | | 14 | O59P catEPO-HL-Fc | 11.057 | 98.94 |
| 6 | C165R catEPO-C1a-Fc | | 10.935 | 99.06 | | 15 | C165R catERO-HL-Fc | 11 .061 | 98.55 |
| 7 | wt catEPO-C1 b-Fc | | 10.922 | 97.95 | | | | | |
| 8 | C59P catEPO-C1 b-Fc | | 10.948 | 99.23 | | | | | |
| 9 | C165R catEPO-C1 b-Fc | | 10.943 | 99 | | | | | |

In Table 8, the retention times of the main peaks showed similar values for all cat EPO-Fc variants. Therefore, it was strongly suggested that cat EPO-Fc variants with C0 type and HL type hinges not containing cysteine have molecular weights similar to those of variants with C2 type, C1a type, and C1b type hinges containing cysteine(s), namely, dimer formation.

From Fig. 4, it was found that the sub-peaks on the left side of the main peak in SEC analysis, which are considered to be aggregates and associations, decrease compared to cat EPO-Fc with wild-type EPO, in cat EPO-Fc variants with C2 type, C1a type, and C1b type hinges, when the C165R or C59 mutation is added to the EPO region in combination with the hinge mutation. Fig. 4F is a chromatogram showing the merged results of the SEC analysis of the cat EPO-Fc fusion proteins: 1. wt cat EPO-C2-Fc, 2. C59P cat EPO-C2-Fc, and 3. C165R cat EPO-C2-Fc (Table 8, 1 - 3). Magnification of the merged chromatograms revealed that 3. C165R cat EPO-C2-Fc had the fewest subpeaks.

Particularly, since cat EPO-Fc with a C165R mutation in the EPO region and a C2 type hinge has few subpeaks, it was found that this variant has good properties.

As described above, it was found that the cat EPO-Fc evaluated in Example 3 mainly forms dimers in all variants, and that the rate of aggregation changes depending on the number of cysteines in the EPO region and the hinge region, which in turn causes a difference in the yield of undenatured proteins after purification. Particularly, in the cat EPO-Fc variant having C165R in the EPO region and having a C2 type hinge region, excessive multimerization such as aggregation tends to be suppressed, and it was found that the protein after purification was a dimer and was superior in both purity and yield.

Example 4. Construction of expression plasmids for fusion proteins of cat EPO and His tag, and mouse EPO and His tag

Expression plasmids for a fusion protein of cat EPO and His tag (hereinafter to be referred to as cat EPO-His or cat EPO-His) and a fusion protein of mouse EPO and His tag (hereinafter to be referred to as mouse EPO-His or mouse EPO-His) to be used as control products of the cat EPO-Fc protein and the like in various experiments were constructed. The detail is shown below.

First, a forward primer that anneals to the N-terminal side of cat EPO and a reverse primer that anneals to the C-terminal side and encodes a His tag (GAAHHHHHHHHH (SEQ ID NO: 14)) containing a linker were designed. Then, 3 types (wild type, C59P, C165R) of DNA fragments of the entire cat EPO-His region were prepared by a PCR method using the designed primers and pCAG/C2 cat wtEPO-Fc, pCAG/C2 C59Pcat EPO-Fc, pCAG/C2 C165R cat EPO-Fc constructed in Example 1 and Example 2 as templates.

Next, the amino acid sequence of mouse EPO-His with a His tag (GAAHHHHHHHHH) containing a linker and attached to the C-terminal side of the amino acid sequence (SEQ ID NO: 15) of mouse EPO registered in GenBank: NM_007942.2 was determined. Gene synthesis was performed by GenScript Japan Inc. based on the amino acid sequence to obtain DNA fragments of all regions of mouse EPO-His. Then, plasmid pCAG was cleaved with restriction enzymes SbfI and EcoRV (both New England Biolabs) to prepare a plasmid fragment opened directly under the CAG promoter. Finally, using the In-Fusion kit, DNA fragments of the three types of cat EPO-His (wild-type, C59P, C165R) and mouse EPO-His were each ligated to the plasmid fragments of pCAG to construct a total of four types of EPO-His plasmids: pCAG/cat wtEPO-His, pCAG/C59P cat EPO-His, pCAG/C165R cat EPO-His, pCAG/mouse wtEPO-His. Use of the In-Fusion kit, and subsequent transformation and culture of Escherichia coli DH5α, extraction of plasmid, and determination of base sequence were performed in the same manner as the method described in Example 1, and construction of the plasmid of interest was confirmed.

wild-type mouse EPO

MGVPERPTLLLLLSLLLIPLGLPVLCAPPRLICDSRVLERYILEAKEAENVTMGCAEGPRLSE

NITVPDTKVNFYAWKRMEVEEQAIEVWQGLSLLSEAILQAQALLANSSQPPETLQLHIDKAIS

GLRSLTSLLRVLGAQKELMSPPDTTPPAPLRTLTVDTFCKLFRVYANFLRGKLKLYTGEVCRR

GDR (SEQ ID NO: 15)

Example 5. Expression of protein by transfection of cat EPO-His, mouse EPO-His expression plasmids into mammalian cells and purification thereof

Using the ExpiCHO™ Expression System Kit (Gibco), which includes cells, medium, and transfection reagent and according to the Max Titer protocol of the kit, each of the pCAG/cat wtEPO-His, pCAG/C59P cat EPO-His, pCAG/C165R cat EPO-His, pCAG/mouse wtEPO-His obtained in Example 4 was introduced into Expi CHO-S™ cells and cultured for 7 days to allow for protein expression. The culture supernatant (30 mL) after the transfection was clarified through a 0.22 μm filter Millex(R)-GP (Merck Millipore). The culture supernatant was added to a column filled with His Trap HP (GE Healthcare Lifesciences), and affinity purification of His tag protein was performed by imidazole gradient elution. Thereafter, gel filtration chromatography using a Superdex 200 gel filtration column (GE Healthcare Lifesciences) and D-PBS(-) as a buffer was further performed to obtain a purified protein. Using a spectrophotometer, the absorption of the purified protein at 280 nm was measured, and the concentration of the purified protein was calculated from the obtained value and using the absorption coefficient calculated by the method of PACE et al. (Protein Science (1995); 4, 2411-2423).

Example 6. Evaluation of in vitro activity of EPO region-, hinge region-altered cat EPO-Fc by using TF-1 cells

Using TF-1 cell, which is a human erythroleukemia cell line exhibiting human EPO-dependent cell proliferation, the biological activity of 15 kinds of cat EPO-Fc prepared in Example 3, 3 kinds of cat EPO-His prepared in Example 5, and purchased human EPO (PeproTech) was evaluated in vitro. The detail is shown below.

After washing BaF3/cat EPOR and TF-1 cells three times with RPMI1640 medium (Thermo Fisher Scientific) containing 10% FBS (Thermo Fisher Scientific) and 100 U/mL penicillin/streptomycin (Thermo Fisher Scientific), the number of cells was adjusted with the same medium to $4 \times 10^5$ cells/mL and 50 μL each was seeded in each well of a 96 well-plate (CORNING). Using the same medium as a diluent, 8 concentrations of 4-fold dilution series of ligands (various type of EPO-Fc, EPO-His, EPO) from 13.16 nM were prepared and 50 μL each thereof was dispensed to each well of the 96-well plate where the cells were seeded. The cells were cultured for 3 days under 37°C, 5% $CO_2$ conditions, Cell Counting Kit-8 (DOJINDO LABORATORIES) was added at 10 μL/well, after culturing for 3 hr, the absorbance at 450 nm (reference wavelength 620 nm) was measured again, and the biological activities of various ligands were evaluated. The results are shown in Fig. 5.

Cat EPO-His, which is a monomer, has a clearly lower activity to proliferate TF-1 cells than human EPO. This seems to be due to the difference in affinity between human EPOR and cat EPO expressed by TF-1 cells. However, cat EPO-Fc clearly shows higher activity than cat EPO-His. In particular, it was confirmed that a mutant with C59P or C165R mutation in the EPO region and a variant with C2 type or C1a type mutation in the hinge region tend to show higher activity than other cat EPO-Fc.

In particular, the activity of a cat EPO-Fc variant with C165R mutation in the EPO region and a C2 type hinge region is high.

Example 7. Construction of expression plasmid for Fc-altered cat EPO-Fc

In Example 3 and Example 6, cat EPO-Fc with C165R mutation in the EPO region and C2 mutation in the hinge region tended to show improved properties and physiological activity. In order to confirm whether the properties of the C165R mutation in the EPO region and the C2 mutation in the hinge region are not impaired in cat EPO-Fc with mutation introduced into wild-type Fc, a cat EPO-Fc expression plasmid was constructed in which a mutation was also introduced into the Fc region. The sequence characteristics of the EPO region, hinge region, and Fc region of the newly constructed cat EPO-Fc variant are shown in Table 9. The detail of plasmid construction is described below.

A primer was designed in which a mutation of wild-type cat Fc DNA was annealed to the site where it is to be introduced and encoding an amino acid sequence characteristic of the Fc variant shown in SEQ ID NO: 3 (to be referred to as CFV3). Then, using pCAG/C2 C165R cat EPO-Fc constructed in Example 2 as templates, a DNA fragment in which a mutation was introduced was amplified by the PCR method using the designed primers, and the amplified DNA fragment was ligated using the In-Fusion kit to construct expression plasmid pCAG/C2 C165R cat EPO-CFV3.

A primer was designed in which a mutation of CFV3 DNA was annealed to the site where it is to be introduced and encoding an amino acid sequence characteristic of the Fc variant shown in SEQ ID NO: 4 (to be referred to as siCFV3). Then, using pCAG/C2 C165R cat EPO-CFV3 as templates, a DNA fragment in which a mutation was introduced was amplified by the PCR method using the designed primers, and the amplified DNA fragment was ligated using the In-Fusion kit to construct expression plasmid pCAG/C2 C165R cat EPO-siCFV3.

Primer to amplify only the Fc region was designed, and a DNA fragment of siCFV3 was prepared by a PCR method using pCAG/C2 C165R cat EPO-siCFV3 as a template. In addition, primers that amplify the EPO region and the EPO to the hinge region were respectively designed, and DNA fragments of wild-type cat EPO containing a C2-type hinge region (cat wtEPO-C2 hinge) and C165R type cat EPO without a hinge region (cat C165R EPO) were respectively prepared by a PCR method using pCAG/C2 cat wtEPO-Fc constructed in Example 1 and pCAG/HL C165R cat EPO-Fc constructed in Example 2 as templates. Finally, EPO (cat wtEPO-C2 hinge, cat C165R EPO) prepared by the PCR method and Fc (siCFV3) DNA fragments and a plasmid fragment of pCAG prepared by the restriction enzyme treatment (SbfI and EcoRV) in Example 1 were linked using the In-Fusion kit to construct pCAG/C2 cat wtEPO-siCFV3 and pCAG/HL C165R cat EPO-siCFV3. Use of the In-Fusion kit, and subsequent transformation and culture of Escherichia coli DH5α, extraction of plasmid, and determination of base sequence were performed in the same manner as the method described in Example 1, and construction of the plasmid of interest was confirmed.

cat Fc variant CFV3

PPEMLGGPSIFIFPPKPKDTLSISRTPEVTCLVVDLGPDDSDVQITWFVDNTQVYTAKTSPRE
EQFNSTYRVVSVLPILHQDWLKGKEFKCKVNSKSLPSPIERTISKAKGQPHEPQVYVLPPAQE
ELSRNKVSVTCLIKSFHPPDIAVEWEITGQPEPENNYRTTPPQLDSDGTYFVYSKLSVDRSHW
QRGNTYTCSVLHEALHAHHTRKELTQSPGK(SEQ ID NO: 3)

cat Fc variant siCFV3

PPEMRRGPKIFIFPPKPKDTLSISRTPEVTCLVVDLGPDDSDVQITWFVDNTQVYTAKTSPRE
EQFNSTYRVVSVLPILHQDWLKGKEFKCKVNSKSLPSPIERTISKAKGQPHEPQVYVLPPAQE
ELSRNKVSVTCLIKSFHPPDIAVEWEITGQPEPENNYRTTPPQLDSDGTYFVYSKLSVDRSHW
QRGNTYTCSVLHEALHAHHTRKELTQSPGK(SEQ ID NO: 4)

[Table 9]

| Fc-altered cat EPO-Fc | | | |
|---|---|---|---|
| plasmid | EPO sequence | hinge sequence | Fc sequence |
| pCAG/C2 C165P cat EPO-siCFV3 | C1 65R ∗∗ | G2:RKTDHPPGPKPGDCPKCP | siCFV3 |
| pCAG/C2 cat wtEPO-siCFV3 | wild type | C2:RKTDHPPGPKPGDCPKCP | siCFV3 |
| pCAG/C2 C1 65R cat EPO-CFV3 | C1 65R ∗∗ | C2:RKTDHPPGPKPGDCPKCP | CFV3 |

(continued)

| Fc-altered cat EPO-Fc | | | |
|---|---|---|---|
| plasmid | EPO sequence | hinge sequence | Fc sequence |
| pCAG/HL C1 65R cat EPO-siCFV3 | C1 65R ∗∗ | HL:- | siCFV3 |
| *C165R means that the 165th amino acid from the start Met of wild-type cat EPO was changed from Cys to Arg. | | | |

Refer to SEQ ID NO: 11 for the sequence of wild-type EPO. Refer to SEQ ID NO: 3 for the sequence of Fc variant CFV3. Refer to SEQ ID NO: 4 for the sequence of Fc variant siCFV3.

Example 8. Protein expression and purification of Fc-altered cat EPO-Fc

Using the ExpiCHO™ Expression System Kit (Gibco), which includes cells, medium, and transfection reagent and according to the Max Titer protocol of the kit, each of the five kinds of cat EPO-Fc expression plasmids (pCAG/C2 C165R cat EPO-Fc constructed in Example 2 and four kinds of expression plasmids for cat EPO-Fc constructed in Example 7: pCAG/C2 C165R cat EPO-siCFV3, pCAG/C2 cat wtEPO-siCFV3, pCAG/C2 C165R cat EPO-CFV3, pCAG/HL C165R cat EPO-siCFV3) were introduced into Expi CHO-S™ cells and cultured for 7 days to allow for protein expression. The culture supernatant (30 mL) after the transfection was clarified through a 0.22 µm filter Millex(R)-GP (Merck Millipore). The culture supernatant (30 mL) after transfection was clarified through a 0.22 um filter Millex(R)-GP (Merck Millipore). The clarified culture supernatant was purified and analyzed by a method similar to that in Example 3.
Fig. 6 shows the SDS-PAGE analysis and SEC analysis results of the purified protein, and Table 10 shows the yield of the purified protein.

[Table 10]

| protein yield after purification | mg |
|---|---|
| C165R catEPO-C2-siCFV3 | 0.21 |
| C165R catEPO-C2-Fc | 0.192 |
| C165R catEPO-C2-CFV3 | 0.199 |
| C165R catEPO-HL-siCFV3 | 0.213 |
| wt catEPO-C2-siCFV3 | 0.144 |

It was found that even when siCFV3 mutation or CFV3 is further introduced into the Fc region of cat EPO-Fc variant with C165R mutation in the EPO region and with C2 type hinge region as the base, the expressed protein forms a dimer and the protein after purification is good in both purity and yield.

Example 9. Evaluation of in vitro activity of Fc-altered cat EPO-Fc by using TF-1 cells

Using TF-1 cell, which is a human erythroleukemia cell line exhibiting human EPO-dependent cell proliferation, the biological activity of cat EPO-Fc prepared in Example 8 and purchased human EPO (PeproTech) was evaluated in vitro. The detail is shown below.
After washing TF-1 cells three times with RPMI1640 medium (Thermo Fisher Scientific) containing 10% FBS (Thermo Fisher Scientific) and 100 U/mL penicillin/streptomycin (Thermo Fisher Scientific), the number of cells was adjusted with the same medium to $4 \times 10^5$ cells/mL and 50 µL each was seeded in each well of a 96 well-plate (CORNING). Using the same medium as a diluent, 8 concentrations of 4-fold dilution series of ligands (various type of EPO-Fc, EPO-His, EPO) from 13.16 nM were prepared and 50 µL each thereof was dispensed to each well of the 96-well plate where the cells were seeded. The cells were cultured for 3 days under 37°C, 5% $CO_2$ conditions, Cell Counting Kit-8 (DOJINDO LABORATORIES) was added at 10 µL/well, after culturing for 3 hr, the absorbance at 450 nm (reference wavelength 620 nm) was measured again, and the biological activities of various ligands were evaluated. The results are shown in Fig. 7.
It was confirmed by comparison with EPO-Fc variant having wild-type EPO region or having no hinge region that the activity tends to be high even when siCFV3 mutation or CFV3 is further introduced into the Fc region of cat EPO-Fc variant with C165R mutation in the EPO region and with C2 type hinge region.

Example 10. Construction of expression plasmid for cat and mouse EPO receptors

Expression plasmids for cat EPO receptor (hereinafter to be referred to as cat EPOR or cat EPOR) and mouse EPO receptor (hereinafter to be referred to as mouse EPOR or mouse EPOR) necessary for establishing a cell line to be used for the evaluation of the biological activity of cat EPO were constructed. The detail is shown below.

Genes were synthesized based on the amino acid sequence (SEQ ID NO: 16) of cat EPOR registered in GenBank: XM_023245578.1, and the amino acid sequence (SEQ ID NO: 17) of mouse EPOR registered in GenBank: NM_010149.3, DNA fragments of cat EPOR and mouse EPOR were obtained. Then, plasmid pBApo-EF1α Pur (TaKaRa Bio) was cleaved with restriction enzymes XbaI and HindIII (both New England Biolabs) to prepare a plasmid fragment opened directly under the EF1α promoter. Finally, using the In-Fusion kit, and the DNA fragments of the cat EPOR and mouse EPOR were each ligated to the plasmid fragments of pBApo-EF1α Pur to construct expression plasmids of cat and mouse EPOR, namely, pBApo-EF1α Pur/cat EPOR and pBApo-EF1α Pur/mouse EPOR. For use the In-Fusion kit, Escherichia coli DH5α competent cells (TOYOBO) were transformed with the DNA solution after the In-Fusion reaction, according to the method described in the attached manual. The obtained transformant was cultured overnight at 37°C in LB liquid medium containing 100 ug/mL ampicillin, and a plasmid was extracted using the NucleoBond Xtra Midi Kit (Takara Bio Inc.) from Escherichia coli obtained by cultivation. The base sequence of the obtained plasmid was determined by a method known to those skilled in the art, and it was confirmed that the protein of the amino acid sequence of interest was encoded.

cat EPOR

MDHLWAPLWPGVGSLCLLLAGAAWAPPPNPLDPKFESKAALLAARGPEELLCFTERLEDLVCF
WEEAASAGVGPDNYSFFYQLEGEPWKPCSLHQAPTARGAVRFWCSLPTADASSFVPLELRVTA
VSSGAPRYHRIIHINEVVLLDPPAGLLARRADEGGHVVLRWLPPPGAPVASLIRYEVNISSGN
VAGGAQKVEILDGRTECALSNLRGRTRYTFMVRARMAEPSFGGFWSAWSEPASLLTASDLDPL
ILTLSLILVLILLLLAVLALLSHRRTLKQKIWPGIPSPESEFEGLFTTHKGNFQLWLYQNEGC
LWWSPCAPFAEDPPSPLEVLSERCWGATQAAEPGAEEGPLLEPLGSEHTQDTYLVLDKWLLPR
NPPSEDLPRPDGSLDMVAMHKGSEASSCSSALSLKPGPEGALGASFEYTILDPSSQLLRPRAL
PPELPPTPPHIKYLYLMVSDSGISTDYSSGGSQEAQGDSSTGPYLNPYENSLIPATETSPPSY
VACS(SEQ ID NO: 16)

mouse EPOR

MDKLRVPLWPRVGPLCLLLAGAAWAPSPSLPDPKFESKAALLASRGSEELLCFTQRLEDLVCF
WEEAASSGMDFNYSFSYQLEGESRKSCSLHQAPTVRGSVRFWCSLPTADTSSFVPLELQVTEA
SGSPRYHRIIHINEVVLLDAPAGLLARRAEEGSHVVLRWLPPPGAPMTTHIRYEVDVSAGNRA
GGTQRVEVLEGRTECVLSNLRGGTRYTFAVRARMAEPSFSGFWSAWSEPASLLTASDLDPLIL
TLSLILVLISLLLTVLALLSHRRTLQQKIWPGIPSPESEFEGLFTTHKGNFQLWLLQRDGCLW
WSPGSSFPEDPPAHLEVLSEPRWAVTQAGDPGADDEGPLLEPVGSEHAQDTYLVLDKWLLPRT
PCSENLSGPGGSVDPVTMDEASETSSCPSDLASKPRPEGTSPSSFEYTILDPSSQLLCPRALP
PELPPTPPHLKYLYLVVSDSGISTDYSSGGSQGVHGDSSDGPYSHPYENSLVPDSEPLHPGYV
ACS(SEQ ID NO: 17)

Example 11. Establishment of Ba/F3 cell line expressing cat EPOR or mouse EPOR

In order to obtain cell lines exhibiting cat EPO- and mouse EPO-dependent proliferation, Ba/F3 cell lines expressing cat EPOR and mouse EPOR were respectively established, as described below. The detail is shown below.

The pBApo-EF1α Pur/cat EPOR, pBApo-EF1α Pur/mouse EPOR (10 ug) constructed in Example 10 were collected, mixed with Ba/F3 cells ($0.8 \times 10^7$ cells) suspended in PBS, and pulse was applied using a Gene Pulser (Bio-Rad) at 0.33 kV, a volume of 950 μFD. Ba/F3 cells transfected by an electroporation treatment were cultured overnight in RPMI1640 medium (Thermo Fisher Scientific) containing 0.4 ng/mL mouse interleukin-3 (R&D Systems), 10% Fetal Bovine Serum (hereinafter FBS, Thermo Fisher Scientific), and 100 U/mL penicillin/streptomycin. The wild-type cat EPO-His and mouse EPO-His prepared in Example 5 were added to the cells, and the cells that proliferate in an EPO-His-dependent manner were selected. Two EPO-His final concentrations, 3.29 nM and 13.16 nM, were tested, and cat EPOR gene-introduced cells were selected using cat EPO-His, and mouse EPOR gene-introduced cells were selected using mouse EPO-His.

By the above-mentioned operations, cat EPOR-expressing Ba/F3 cell line (hereinafter BaF3/cat EPOR) and mouse EPOR-expressing Ba/F3 cell line (hereinafter BaF3/mouse EPOR) were established. It was confirmed that the established BaF3/cat EPOR proliferates in a cat EPO-His-dependent manner, and BaF3/mouse EPOR proliferates in a mouse EPO-His-dependent manner.

Example 12. Evaluation of in vitro activity of cat EPO-Fc by using BaF3/mouse EPOR

Whether the cat EPO-Fc (C165R cat EPO-siCFV3) prepared in Example 8 can proliferate the BaF3/mouse EPOR established in Example 11 in a concentration-dependent manner was confirmed. As controls for cat EPO-Fc, the activities of wild-type cat EPO-His and wild-type mouse EPO-His prepared in Example 5, and purchased wild-type human EPO (PeproTech) were also evaluated at the same time. The detail is shown below.

After washing BaF3/mouse EPOR three times with RPMI1640 medium (Thermo Fisher Scientific) containing 10% FBS (Thermo Fisher Scientific) and 100 U/mL penicillin/streptomycin (Thermo Fisher Scientific), the number of cells was adjusted with the same medium to $1.5 \times 10^5$ cells/mL and 50 μL each was seeded in each well of a 96 well-plate (CORNING). Using the same medium as a diluent, 8 concentrations of 4-fold dilution series of ligands (cat EPO-Fc, cat EPO-His, mouse EPO-His, human EPO) from 13.16 nM were prepared and 50 μL each thereof was dispensed to each well of the 96-well plate where the cells were seeded. The cells were cultured for 2 days under 37°C, 5% $CO_2$ conditions, Cell Counting Kit-8 (DOJINDO LABORATORIES) was added at 10 μL/well, after culturing for 2 hr, the absorbance at 450 nm (reference wavelength 620 nm) was measured again, and the biological activities of various ligands were evaluated. The results are shown in Fig. 8.

It was confirmed in vitro that cat EPO-His and cat EPO-Fc act on mouse EPOR in a concentration dependent manner. Cat EPO-Fc was considered to exhibit physiological activity also in in vivo experiments in which it is administered to individual mice.

Example 13. Evaluation of in vitro activity of cat EPO-Fc by using BaF3/cat EPOR

Whether the cat EPO-Fc (C165R cat EPO-siCFV3) prepared in Example 8 can proliferate the BaF3/cat EPOR established in Example 11 in a concentration-dependent manner was confirmed. As controls for cat EPO-Fc, the activities of wild-type cat EPO-His prepared in Example 5 and purchased wild-type human EPO (PeproTech) were also evaluated at the same time. The detail is shown below.

After washing BaF3/cat EPOR three times with RPMI1640 medium (Thermo Fisher Scientific) containing 10% FBS (Thermo Fisher Scientific) and 100 U/mL penicillin/streptomycin (Thermo Fisher Scientific), the number of cells was adjusted with the same medium to $1.5 \times 10^5$ cells/mL and 50 μL each was seeded in each well of a 96 well-plate (CORNING). Using the same medium as a diluent, 8 concentrations of 4-fold dilution series of ligands (cat EPO-Fc, cat EPO-His, human EPO) from 13.16 nM were prepared and 50 μL each thereof was dispensed to each well of the 96-well plate where the cells were seeded. The cells were cultured for 2 days under 37°C, 5% $CO_2$ conditions, Cell Counting Kit-8 (DOJINDO LABORATORIES) was added at 10 μL/well, after culturing for 2 hr, the absorbance at 450 nm (reference wavelength 620 nm) was measured again, and the biological activities of various ligands were evaluated. The results are shown in Fig. 9.

It was confirmed that cat EPO-Fc proliferates BaF3/cat EPOR cells in a concentration dependent manner. In addition, it was shown that cat EPO-Fc has higher activity than cat EPO-His. Cat EPO-Fc was considered to exhibit physiological activity also in in vivo experiments in which it is administered to individual cats.

[Industrial Applicability]

The EPO-Fc fusion protein of the present invention is superior in the property and activity. According to the present invention, the EPO-Fc fusion protein can be obtained in a high yield.

This application is based on a patent application No. 2020-101237 filed in Japan (filing date: June 10, 2020), the contents of which are incorporated in full herein.

**Claims**

1. A fusion protein comprising an erythropoietin polypeptide fused to the Fc region of a dog or cat-derived IgG.

2. The fusion protein according to claim 1, wherein the erythropoietin polypeptide is fused to the Fc region via a hinge region of the IgG.

3. The fusion protein according to claim 1 or 2, wherein the erythropoietin polypeptide has at least one mutation, and the mutation is substitution of at least one cysteine in a wild-type sequence thereof with arginine or proline.

4. The fusion protein according to claim 3, wherein the erythropoietin polypeptide having at least one mutation is derived from cat, and has the amino acid sequence shown in SEQ ID NO: 9 or 10.

5. The fusion protein according to any one of claims 2 to 4, wherein the hinge region has at least one mutation, and the mutation is substitution of at least one cysteine in a wild-type sequence thereof with glycine.

6. The fusion protein according to claim 5, wherein the hinge region having at least one mutation is derived from cat, and has the amino acid sequence shown in any of SEQ ID NO: 5 - 8.

7. The fusion protein according to any one of claims 1 to 6, wherein the Fc region has an amino acid alteration.

8. The fusion protein according to claim 7, wherein the Fc region is derived from dog IgG.

9. The fusion protein according to claim 7, wherein the Fc region is derived from cat IgG.

10. The fusion protein according to claim 8, wherein the amino acid alteration in the Fc region comprises at least one selected from the group consisting of

    (i) substitution of the 252-position leucine with tyrosine or threonine,
    (ii) substitution of the 254-position alanine with threonine,
    (iii) substitution of the 256-position threonine with glutamic acid,
    (iv) substitution of the 308-position isoleucine with proline,
    (v) substitution of the 428-position methionine with leucine,
    (vi) substitution of the 433-position histidine with leucine,
    (vii) substitution of the 434-position asparagine with alanine, serine, tyrosine or phenylalanine,
    (viii) substitution of the 436-position tyrosine with threonine,
    (ix) substitution of the 438-position glutamine with arginine, and
    (x) substitution of the 440-position serine with glutamic acid (wherein the numbering of amino acids in the Fc region is based on EU Index of Kabat using Fc region of human antibody as the standard).

11. The fusion protein according to claim 10, wherein the amino acid alterations in the Fc region are

    (i) substitution of the 434-position asparagine with alanine,
    (ii) substitution of the 436-position tyrosine with threonine,
    (iii) substitution of the 438-position glutamine with arginine, and
    (iv) substitution of the 440-position serine with glutamic acid.

12. The fusion protein according to claim 10, wherein the amino acid alterations in the Fc region are

    (i) substitution of the 428-position methionine with leucine,
    (ii) substitution of the 434-position asparagine with alanine,
    (iii) substitution of the 436-position tyrosine with threonine,
    (iv) substitution of the 438-position glutamine with arginine, and
    (v) substitution of the 440-position serine with glutamic acid.

**13.** The fusion protein according to claim 10, wherein the amino acid alteration in the Fc region are

(i) substitution of the 428-position methionine with leucine,
(ii) substitution of the 434-position asparagine with alanine,
(iii) substitution of the 438-position glutamine with arginine, and
(iv) substitution of the 440-position serine with glutamic acid.

**14.** The fusion protein according to claim 10, wherein the amino acid alteration in the Fc region are

(i) substitution of the 252-position leucine with tyrosine,
(ii) substitution of the 254-position alanine with threonine, and
(iii) substitution of the 256-position threonine with glutamic acid.

**15.** The fusion protein according to claim 10, wherein the amino acid alteration in the Fc region are

(i) substitution of the 428-position methionine with leucine, and
(ii) substitution of the 434-position asparagine with serine.

**16.** The fusion protein according to claim 10, wherein the amino acid alteration in the Fc region are

(i) substitution of the 308-position isoleucine with proline, and
(ii) substitution of the 434-position asparagine with tyrosine.

**17.** The fusion protein according to claim 10, wherein the amino acid alteration in the Fc region are

(i) substitution of the 252-position leucine with threonine,
(ii) substitution of the 254-position alanine with threonine,
(iii) substitution of the 256-position threonine with glutamic acid,
(iv) substitution of the 433-position histidine with leucine, and
(v) substitution of the 434-position asparagine with phenylalanine.

**18.** The fusion protein according to claim 9, wherein the amino acid alteration in the Fc region includes at least one selected from the group consisting of

(i) substitution of the 252-position serine with tyrosine or threonine,
(ii) substitution of the 254-position serine with threonine,
(iii) substitution of the 256-position threonine with glutamic acid,
(iv) substitution of the 259-position valine with isoleucine,
(v) substitution of the 308-position isoleucine with proline or phenylalanine,
(vi) substitution of the 428-position serine with leucine,
(vii) substitution of the 433-position histidine with leucine,
(viii) substitution of the 434-position serine with alanine, tyrosine or phenylalanine,
(ix) substitution of the 436-position histidine with threonine,
(x) substitution of the 438-position glutamine with arginine,
(xi) substitution of the 440-position serine with glutamic acid,
(xii) substitution of the 235-position leucine with arginine,
(xiii) substitution of the 236-position glycine with arginine, and
(xiv) substitution of the 239-position serine with lysine (wherein the numbering of amino acids in the Fc region is based on EU Index of Kabat using Fc region of human antibody as the standard).

**19.** The fusion protein according to claim 18, wherein the amino acid alterations in the Fc region are

(i) substitution of the 434-position serine with alanine,
(ii) substitution of the 436-position histidine with threonine,
(iii) substitution of the 438-position glutamine with arginine, and
(iv) substitution of the 440-position serine with glutamic acid.

**20.** The fusion protein according to claim 18, wherein the amino acid alterations in the Fc region are

(i) substitution of the 428-position serine with leucine,
(ii) substitution of the 434-position serine with alanine,
(iii) substitution of the 436-position histidine with threonine,
(iv) substitution of the 438-position glutamine with arginine, and
(v) substitution of the 440-position serine with glutamic acid.

21. The fusion protein according to claim 18, wherein the amino acid alterations in the Fc region are

(i) substitution of the 428-position serine with leucine,
(ii) substitution of the 434-position serine with alanine,
(iii) substitution of the 438-position glutamine with arginine, and
(iv) substitution of the 440-position serine with glutamic acid.

22. The fusion protein according to claim 18, wherein the amino acid alterations in the Fc region are

(i) substitution of the 252-position serine with tyrosine,
(ii) substitution of the 254-position serine with threonine, and
(iii) substitution of the 256-position threonine with glutamic acid.

23. The fusion protein according to claim 18, wherein the amino acid alterations in the Fc region are

(i) substitution of the 308-position isoleucine with proline, and
(ii) substitution of the 434-position serine with tyrosine.

24. The fusion protein according to claim 18, wherein the amino acid alterations in the Fc region are

(i) substitution of the 259-position valine with isoleucine,
(ii) substitution of the 308-position isoleucine with phenylalanine, and
(iii) substitution of the 428-position serine with leucine.

25. The fusion protein according to claim 18, wherein the amino acid alterations in the Fc region are

(i) substitution of the 252-position serine with threonine,
(ii) substitution of the 254-position serine with threonine,
(iii) substitution of the 256-position threonine with glutamic acid,
(iv) substitution of the 433-position histidine with leucine, and
(v) substitution of the 434-position serine with phenylalanine.

26. The fusion protein according to claim 18, wherein the amino acid alterations in the Fc region are

(vi) substitution of the 428-position serine with leucine,
(viii) substitution of the 434-position serine with alanine,
(x) substitution of the 438-position glutamine with arginine,
(xi) substitution of the 440-position serine with glutamic acid,
(xii) substitution of the 235-position leucine with arginine,
(xiii) substitution of the 236-position glycine with arginine, and
(xiv) substitution of the 239-position serine with lysine.

27. The fusion protein according to claim 7, wherein the Fc region of IgG having the amino acid alteration is derived from cat, and has the amino acid sequence shown in SEQ ID NO: 3 or 4.

28. A pharmaceutical composition comprising the fusion protein according to any one of claims 1 to 27.

29. The pharmaceutical composition according to claim 28 for the treatment of a hematopoiesis disease or hematopoiesis failure.

[Fig. 1]

[Fig. 2]

1 wt catEPO-C2-Fc       9 C165R catEPO-C1b-Fc
2 C59P catEPO-C2-Fc     10 Wt catEPO-C0-Fc
3 C165R catEPO-C2-Fc    11 C59P catEPO-C0-Fc
4 wt catEPO-C1a-Fc      12 C165R catEPO-C0-Fc
5 C59P catEPO-C1a-Fc    13 wt catEPO-HL-Fc
6 C165R catEPO-C1a-Fc   14 C59P catEPO-HL-Fc
7 wt catEPO-C1b-Fc      15 C165R catEPO-HL-Fc
8 C59P catEPO-C1b-Fc     M Marker

[Fig. 3]

non-reducing SDS-PAGE

reducing SDS-PAGE

1 wt catEPO–C2–Fc      9 C165R catEPO–C1b–Fc

2 C59P catEPO–C2–Fc      10 Wt catEPO–C0–Fc

3 C165R catEPO–C2–Fc      11 C59P catEPO–C0–Fc

4 wt catEPO–C1a–Fc      12 C165R catEPO–C0–Fc

5 C59P catEPO–C1a–Fc      13 wt catEPO–HL–Fc

6 C165R catEPO–C1a–Fc      14 C59P catEPO–HL–Fc

7 wt catEPO–C1b–Fc      15 C165R catEPO–HL–Fc

8 C59P catEPO–C1b–Fc      M Marker

[Fig. 4A]

1 wt catEPO-C2-Fc

2 C59P catEPO-C2-Fc

3 C165R catEPO-C2-Fc

EP 4 166 574 A1

[Fig. 4B]

4 wt catEPO-C1a-Fc

5 C59P catEPO-C1a-Fc

6 C165R catEPO-C1a-Fc

[Fig. 4C]

7 wt catEPO-C1b-Fc

8 C59P catEPO-C1b-Fc

9 C165R catEPO-C1b-Fc

[Fig. 4D]

[Fig. 4E]

13 wt catEPO-HL-Fc

14 C59P catEPO-HL-Fc

15 C165R catEPO-HL-Fc

EP 4 166 574 A1

EP 4 166 574 A1

EP 4 166 574 A1

[Fig. 5]

[Fig. 6A]

kDa
-250
-130
-100
-70
-55
-35

A: C165R catEPO-C2-siCFV3
B: C165R catEPO-C2-Fc
C: C165R catEPO-C2-CFV3
D: C165R catEPO-HL-siCFV3
E: wt catEPO-C2-siCFV3
M: Marker

non-reducing        reducing

SDS-PAGE analysis of purified protein

[Fig. 6B]

SEC analysis of Fc-altered cat EPO-Fc protein

| peak No. | C165R catEPO-C2-siCFV3 | | C165R catEPO-C2-Fc | | C165R catEPO-C2-CFV3 | | C165R catEPO-HL-siCFV3 | | wt catEPO-C2-siCFV3 | |
|---|---|---|---|---|---|---|---|---|---|---|
| | retention time (min) | area (%) | retention time (min) | area (%) | retention time (min) | area (%) | retention time (min) | area (%) | retention time (min) | area (%) |
| Peak_1 | - | - | 3.123 | 0.09 | 3.209 | 0.08 | 3.217 | 0.07 | 3.223 | 0.09 |
| Peak_2 | 8.775 | 0.05 | 8.772 | 0.01 | 8.788 | 0.05 | - | - | 8.749 | 0.13 |
| Peak_3 | - | - | - | - | 9.717 | 0.03 | 9.656 | 0.34 | - | - |
| Peak_4 | 10.264 | 0.20 | 10.278 | 0.27 | 10.289 | 0.17 | 10.394 | 0.85 | 10.263 | 0.49 |
| Peak_5 | 11.585 | 99.73 | 11.621 | 99.61 | 11.633 | 99.66 | 11.887 | 98.73 | 11.574 | 99.27 |
| Peak_6 | 16.962 | 0.01 | 16.953 | 0.01 | 16.952 | 0.01 | 16.948 | 0.01 | 16.956 | 0.01 |
| Peak_7 | 17.516 | 0.01 | 17.508 | 0.01 | 17.516 | 0.01 | 17.49 | 0.01 | 17.509 | 0.01 |

[Fig. 7]

[Fig. 8]

[Fig. 9]

## INTERNATIONAL SEARCH REPORT

| International application No. |
| --- |
| PCT/JP2021/021972 |

**A. CLASSIFICATION OF SUBJECT MATTER**
C07K 19/00(2006.01)i; A61K 38/22(2006.01)i; A61K 47/68(2017.01)i; A61P
7/06(2006.01)i; C12N 15/12(2006.01)i; C12N 15/13(2006.01)i; C12N
15/62(2006.01)i; C12P 21/02(2006.01)i
FI: C07K19/00; A61P7/06; A61K38/22; A61K47/68; C12N15/13; C12N15/12;
C12P21/02 Z; C12N15/62 Z ZNA

According to International Patent Classification (IPC) or to both national classification and IPC

**B. FIELDS SEARCHED**

Minimum documentation searched (classification system followed by classification symbols)
C07K19/00; A61K38/22; A61K47/68; A61P7/06; C12N15/12; C12N15/13;
C12N15/62; C12P21/02

Documentation searched other than minimum documentation to the extent that such documents are included in the fields searched
Published examined utility model applications of Japan           1922-1996
Published unexamined utility model applications of Japan         1971-2021
Registered utility model specifications of Japan                 1996-2021
Published registered utility model applications of Japan         1994-2021

Electronic data base consulted during the international search (name of data base and, where practicable, search terms used)
JSTPlus/JMEDPlus/JST7580 (JDreamIII); CAplus/MEDLINE/EMBASE/BIOSIS (STN);
GenBank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2018/226747 A1 (KINDRED BIOSCIENCES, INC.) 13 December 2018 (2018-12-13) claims, abstract, example 1 | 1-29 |
| Y | WO 2018/073185 A1 (VETOQUINOL SA) 26 April 2018 (2018-04-26) claims, page 1, line 30 to page 4, line 9, page 8, lines 14-26, page 19, lines 3-6, SEQ ID NO: 1 | 1-29 |
| Y | JP 2008-504008 A (MERCK PATENT GMBH) 14 February 2008 (2008-02-14) claims, abstract, paragraphs [0017], [0054], [0088] | 3-29 |

☒ Further documents are listed in the continuation of Box C.   ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 24 August 2021 (24.08.2021) | 31 August 2021 (31.08.2021) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2021/021972 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 2009-524415 A (NOVAGEN HOLDING CORPORATION) 02 July 2009 (2009-07-02) claims, abstract, paragraphs [0020], [0021] | 5-29 |
| A | RANDOLPH, J. F. et al., "Expression, bioactivity, and clinical assessment of recombinant feline erythropoietin", American Journal of Veterinary Research, 2004, vol. 65, no. 10, pp. 1355-1366, entire text | 1-29 |
| A | WEN, D. et al., "Erythropoietin Structure-Function Relationships: High Degree of Sequence Homology Among Mammals", Blood, 1993, vol. 82, no. 5, pp. 1507-1516, entire text | 1-29 |
| P, Y | WO 2020/116560 A1 (THERAPEUTICS INC.) 11 June 2020 (2020-06-11) claims, abstract | 1-29 |

Form PCT/ISA/210 (continuation of second sheet) (January 2015)

International application No.

PCT/JP2021/021972

| Patent Documents referred in the Report | Publication Date | Patent Family | Publication Date |
|---|---|---|---|
| WO 2018/226747 A1 | 13 Dec. 2018 | JP 2020-522263 A | |
| WO 2018/073185 A1 | 26 Apr. 2018 | US 2020/0181258 A1 | |
| JP 2008-504008 A | 14 Feb. 2008 | JP 2012-107042 A<br>WO 2005/063808 A1<br>claims, abstract,<br>page 6, line 32 to<br>page 7, line 18, page<br>21, line 21 to page<br>22, line 15, page 34,<br>lines 6-19<br>US 2005/0192211 A1<br>US 2005/0202538 A1<br>US 2009/0092607 A1 | |
| JP 2009-524415 A | 02 Jul. 2009 | JP 2013-66477 A<br>WO 2007/085084 A1<br>claims, abstract,<br>paragraphs [0038],<br>[0039]<br>JP 2016-6116 A<br>US 2007/0178112 A1<br>US 2009/0297522 A1<br>US 2010/0098716 A1<br>US 2010/0099145 A1<br>US 2013/0224198 A1<br>US 2017/0114110 A1<br>US 2019/0070307 A1<br>EP 2450373 A2 | |
| WO 2020/116560 A1 | 11 Jun. 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9902709 A
- WO 2002060919 A
- WO 2012083370 A
- EP 2154157 A
- US 20070141052 A
- WO 2000042072 A
- WO 2006020114 A
- WO 2006031370 A
- WO 2010033279 A
- WO 2006053301 A
- WO 2009086320 A
- JP 2020101237 A

### Non-patent literature cited in the description

- **YEUNG YA et al.** *J. Immunol.,* 2009, vol. 182, 7663-71
- **DATTA-MANNAN A et al.** *J. Biol. Chem.,* 2007, vol. 282, 1709-17
- **DALL'ACQUA WF et al.** *J. Immunol.,* 2002, vol. 169, 5171-80
- **TANG et al.** *Vet. Immunol. Immunopathol.,* 2001, vol. 80 (3-4), 259-270
- **KANAI, T.H. et al.** Identification of two allelic IgG1 C(H)coding regions (Cgamma1) of cat. *Vet. Immunol. Immunopathol.,* 2000, vol. 73 (1), 53-62
- **STRIETZEL, C.J. et al.** In Vitro functional characterization of feline IgGs. *Vet. Immunol. Immunopathol.,* 2014, vol. 158 (3-4), 214-233
- **GHETIE et al.** *Immunol. Today,* 1997, vol. 18 (12), 592-598
- **RAGHAVAN et al.** *Immunity,* 1994, vol. 1, 303-315
- **KABAT et al.** Sequences of Proteins of Immunological Interest. Public Health Service, National Institutes of Health, 1991
- *Current Pharmaceutical Biotechnology,* 2016, vol. 17, 1298-1314
- *Proc Natl Acsd Sci USA,* 1984, vol. 81, 5662-5666
- **SAMBROOK et al.** Molecular Cloning A Laboratory Manual. Cold Spring Harbor Laboratory Press, 1989
- *Annu. Rev. Biophys. Biomol. Struct.,* 2006, vol. 35, 225-249
- *Proc. Natl. Acad. Sci. U.S.A.,* 2003, vol. 100 (11), 6353-6357
- *Drug Metab Dispos.,* January 2007, vol. 35 (1), 86-94
- *Int Immunol.,* December 2006, vol. 18 (12), 1759-69
- *J Biol Chem.,* 02 March 2001, vol. 276 (9), 6591-604
- *J Biol Chem.,* 2007, vol. 282 (3), 1709-17
- *J Immunol.,* 2002, vol. 169 (9), 5171-80
- *J Immunol.,* 2009, vol. 182 (12), 7663-71
- *Molecular Cell,* April 2001, vol. 7, 867-877
- *Nat Biotechnol.,* July 1997, vol. 15 (7), 637-40
- *Nat Biotechnol.,* October 2005, vol. 23 (10), 1283-8
- *Proc Natl Acad Sci U S A.,* 05 December 2006, vol. 103 (49), 18709-14
- *Mol Cell Biol.,* 1988, vol. 8, 466-472
- *J. Exp. Med.,* 1995, vol. 108, 94
- **VALLE et al.** *Nature,* 1981, vol. 291, 338-340
- Remington's Pharmaceutical Science. Mark Publishing Company
- **PACE et al.** *Protein Science,* 1995, vol. 4, 2411-2423